# EUROPEAN PATENT APPLICATION

(11) **EP 4 317 424 A1**
(43) Date of publication of application: **07.02.2024**
(21) Application number: 22780206.3
(22) Date of filing: 17.03.2022
(51) Int. Cl.: C12N 5/10, C12N 15/11, C12N 15/54, C12N 15/63, C12N 15/864

(54) **VECTOR, METHOD FOR PREPARING LINEAR COVALENT BOND CLOSED DNA USING SAME, PARVOVIRUS VECTOR PREPARATION METHOD, AND PARVOVIRUS VECTOR PRODUCING CELL**

(30) Priority: 29.03.2021 JP 2021054930; 29.03.2021 JP 2021054970
(71) Applicant: Kaneka Corporation, Osaka-shi, Osaka 530-8288 (JP)
(72) Inventor: NISHI, Teruyuki, Takasago-shi, Hyogo 676-8688 (JP); MAEDA, Hirofumi, Takasago-shi, Hyogo 676-8688 (JP); KITANO, Mitsuaki, Takasago-shi, Hyogo 676-8688 (JP)
(74) Representative: Hoffmann Eitle
(86) International application number: PCT/JP2022/012427
(87) International publication number: WO 2022/209987

(57) **Abstract**

A vector that includes a nucleic acid sequence encoding protelomerase, a pair of nucleic acid sequences recognized by the protelomerase, and a nucleic acid sequence located between the pair of nucleic acid sequences and encoding a target protein. A linear covalently closed DNA production method using the vector, and a parvovirus vector production method that includes: a gene introduction step of introducing, into a first host, a vector including a nucleic acid sequence encoding protelomerase, a pair of nucleic acid sequences recognized by the protelomerase, and a nucleic acid sequence encoding a protein; and a transfection step of transfecting a second host with a linear covalently closed DNA obtained in the gene introduction step. A parvovirus-vector-producing cell. The parvovirus vector is a parvovirus vector that is obtained using a linear covalently closed DNA and expresses a nucleic acid sequence encoding a protein, where the number of vector genomes of sequences other than the nucleic acid sequence encoding the protein relative to the number of vector genomes of the nucleic acid sequence encoding the protein, as quantified by quantitative PCR, is 10% or less in a culture supernatant of the parvovirus-vector-producing cells, or 1% or less in a cell solution of the parvovirus-vector-producing cells.

## Description

### Technical Field

The present invention relates to a vector, a linear covalently closed DNA production method using the vector, a parvovirus vector production method, and a parvovirus-vector-producing cell.

### Background

In gene delivery technologies for tissues or cells, DNA vectors are widely used as non-viral vectors.

As the DNA vectors, double-stranded circular plasmid DNA vectors, which can be produced by simple production methods, are generally used. However, some concerns still remain with plasmid DNA vectors, such as immunogenicity caused by residual nucleic acid sequences derived from a host (e.g., a gene encoding an antibiotic resistant protein and a replication factor sequence), and expression attenuation of a target protein within a cell (NPL 1).

Looking at another aspect, a linear double-stranded DNA vector, ends of which are not closed, can be simply produced using PCR etc., but is quickly decomposed inside and outside cells due to insufficient nuclease resistance.

In order to solve the above-described problems, the following is reported: a double-stranded circular DNA vector, such as a minicircle, in which nucleic acid sequences derived from a host are removed to reduce a size of the DNA vector, and a production method thereof (NPLs 2 and 3); and a production method of a linear covalently closed DNA that is a vector where ends of a double-stranded DNA are each closed with a hairpin structure (PLTs 1 and 2).

The above-described vectors are significantly beneficial because of the following reasons: transfer efficiency of the vectors into cells increases owing to the small size thereof compared to a typical double-stranded circular plasmid DNA vector; risks (e.g., immunogenicity, etc.) are minimized as unnecessary base sequences (e.g., nucleic acid sequences derived from a host, etc.) are not included; high quality gene delivery can be expected; and sustained expression of a target protein within a cell can be expected.

If innovative production techniques for the above-described DNA vectors can be developed, therefore, industrial application over a wide range of fields can be expected.

In a case where minicircles are produced, however, problems may include occurrences of random recombination of a DNA vector into a host genome, or low yield of a DNA vector (NPLs 2 and 3).

In a case where a linear covalently closed DNA is produced by a method using recombinant *E. coli* (PTL 1), moreover, other problems and complications may be caused, such as recombination of an enzyme gene into an *E. coli* genome, essential use of a specific sequence recognized by the enzyme, possible occurrences of random recombination of a DNA vector into a host genome, and a low yield of a DNA vector. Moreover, it has been also reported that a yield of a linear covalently closed DNA is significantly lower than that of a minicircle.

Looking at another aspect, in a case where a linear covalently closed DNA is produced by amplifying DNA to be used as a template in vitro using DNA polymerase and processing with an enzyme (PTL 2), other problems arise, such as an increase in production cost, complication of the processing step, and possible mutation occurred in the process of amplification of the template DNA by the DNA polymerase.

Accordingly, a production method for a linear covalently closed DNA, which is highly efficient and enables simple production, has not been yet known.

Moreover, an adeno-associated virus (AAV) vector that belongs to the Parvoviridae family is one of carriers that are the most actively developed in the field of gene therapies.

A recombinant adeno-associated virus (rAAV) vector is generally produced by simultaneously transfecting HEK293 cells or insect cells with three double-stranded circular plasmid DNA vectors (a vector including a target gene, a vector including a packaging gene, and a vector including an adenovirus helper gene).

It has been known that a gene encoding an antibiotic resistant protein, a replication factor sequence, a packaging gene, or an adenovirus helper gene is erroneously packaged when an rAAV vector is produced using the double-stranded circular plasmid DNA vectors (NPL 4), which leads to concerns, such as possible inclusion of a self-reproduceable AAV (rcAAV) vector as impurities, and immunogenicity due to residual host-derived nucleic acid sequences.

As described above, a double-stranded circular DNA vector, which is called a minicircle, and a linear covalently closed DNA have been known as the DNA vectors. The above-mentioned vectors are expected to enhance transfer efficiency into cells compared to typical double-stranded circular plasmid DNA vectors, to reduce risks (e.g., immunogenicity, etc.), and to achieve high-quality gene delivery.

In a case where a minicircle is produced (NPL 5), however, a yield of a DNA vector is low, and an rAAV vector is erroneously packaged because a homologous recombination sequence and a sequence for purification, which are used for production of a minicircle, remain in between inverted terminal repeat sequences (ITRs).

As a method of producing an rAAV vector, a method of recombining required nucleic acid sequences into genomes of a cell has been reported (PTL 3). However, such a method leaves a possibility of erroneous packaging with sequences near ITRs due to the above-described reason, and an increased risk of simultaneous production and inclusion of a rcAAV vector when all of the nucleic acid sequences are recombined together in a single locus.

As described above, a production method and a parvovirus-vector-producing cell, which are capable of producing a high-quality parvovirus vector in a simple manner at high efficiency using a linear covalently closed DNA that is substantially free from unnecessary sequences, have not been yet known.

### Citation List

### [Patent Literature]

[PTL 1] US Patent No. 9,290,778
[PTL 2] US Patent No. 9,109,250
[PTL 3] Japanese Unexamined Patent Application Publication (JP-A) No. 2020-517238

### [Non-Patent Literature]

[NPL 1] Mol Ther. 2004 10: 269-78
[NPL 2] Gene Ther. 1999 6: 209-18
[NPL 3] Mol Ther. 2012 21: 131-8
[NPL 4] JOURNAL OF VIROLOGY. 2003 Jan: 776-781
[NPL 5] Mol Ther -Nucleic Acids. 2016 5: e355

### Summary of the Invention

### Technical Problem

The present invention aims to solve the above-described various problems existing in the related art, and to achieve the following object. Namely, an object of the present invention is to provide a linear covalently closed DNA production method that can produce a linear covalently closed DNA in a simple manner at high efficiency, a parvovirus vector production method using a linear covalently closed DNA substantially free from unnecessary sequences, where the method can produce a high-quality parvovirus vector in a simple manner at high efficiency, and a parvovirus-vector-producing cell.

### Solution to Problem

The present inventors have diligently conducted research to achieve the above-described object. As a result, the present inventors have gained the following insight. That is, a linear covalently closed DNA production method, which is highly efficient and enables simple production, can be provided using a vector including a nucleic acid sequence encoding protelomerase, a pair of nucleic acid sequences recognized by the protelomerase, and a nucleic acid sequence located between the pair of nucleic acid sequences and encoding a target protein. Moreover, a method of producing a high-quality parvovirus vector, and a parvovirus-vector-producing cell, which are highly efficient and enable simple production using a linear covalently closed DNA substantially free from unnecessary sequences, are provided by a parvovirus vector production method including a gene introduction step and a transfection step, where the gene introduction step includes introducing, into a first host, a vector including a nucleic acid sequence encoding protelomerase, a pair of nucleic acid sequences recognized by the protelomerase, and a nucleic acid sequence located between the pair of nucleic acid sequences and encoding a protein, and the transfection step includes transfecting a second host with the linear covalently closed DNA obtained in the gene introduction step.

The present invention is based upon the above-described insights of the present inventors, and the means for solving the above-described problems are as follows.
<1> A vector including:
   a nucleic acid sequence encoding protelomerase;
   a pair of nucleic acid sequences recognized by the protelomerase; and
   a nucleic acid sequence located between the pair of nucleic acid sequences and encoding a protein.
<2> A linear covalently closed DNA production method, including:
   a gene introduction step of introducing a vector into a host, where the vector includes a nucleic acid sequence encoding protelomerase, a pair of nucleic acid sequences recognized by the protelomerase, and a nucleic acid sequence located between the pair of nucleic acid sequences and encoding a protein.
<3> A parvovirus vector production method, including:
   a gene introduction step of introducing, into a first host, a vector including a nucleic acid sequence encoding protelomerase, a pair of nucleic acid sequences recognized by the protelomerase, and a nucleic acid sequence located between the pair of nucleic acid sequences and encoding a protein; and
   a transfection step of transfecting a second host with a linear covalently closed DNA obtained in the gene introduction step.
<4> A parvovirus-vector-producing cell,
   wherein the parvovirus vector is a parvovirus vector that is obtained using a linear covalently closed DNA and expresses a nucleic acid sequence encoding a protein, and
   wherein the number of vector genomes of sequences other than the nucleic acid sequence encoding the protein relative to the number of vector genomes of the nucleic acid sequence encoding the protein, as quantified by quantitative PCR, is 10 percent (%) or less in a culture supernatant of the parvovirus-vector-producing cells, or 1% or less in a cell solution of the parvovirus-vector-producing cells.

### Advantageous Effects of Invention

According to the present invention, the above-described various problems existing in the related art can be solved; the above-described object can be achieved; and a linear covalently closed DNA production method that can produce a linear covalently closed DNA in a simple manner at high efficiency, a vector used for the linear covalently closed DNA production method, a parvovirus vector production method using a linear covalently closed DNA substantially free from unnecessary sequences, where the method can produce a high-quality parvovirus vector in a simple manner at high efficiency, and a parvovirus-vector-producing cell can be provided.

### Brief Description of the Drawings

[Fig. 1] Fig. 1 is a diagram depicting a vector map of pAAV-Venus_telRL_ara_TelN in Examples 1-1 and 2-1.
[Fig. 2] Fig. 2 is a diagram depicting the evaluation results from electrophoresis of the DNA obtained in Example 1-3.
[Fig. 3] Fig. 3 is a diagram depicting the evaluation results from electrophoresis of the DNA obtained in Example 1-4.
[Fig. 4] Fig. 4 is a diagram depicting a vector map of pAAV-Venus_telRL_IPTG_TelN in Example 1-5.
[Fig. 5] Fig. 5 is a diagram depicting a vector map of pRC2-mi342_telRL_ara_TelN in Example 2-2.
[Fig. 6] Fig. 6 is a diagram depicting a vector map of pHelper_telRL_ara_TelN in Example 2-3.
[Fig. 7] Fig. 7 is a diagram depicting a vector map of pRC2-mi342_telRL_ara_TelN_Helper in Example 2-4.
[Fig. 8] Fig. 8 is a diagram depicting the evaluation results from electrophoresis of the DNA obtained in Example 2-6.
[Fig. 9] Fig. 9 is a diagram depicting the evaluation results from electrophoresis of the DNA obtained in Example 2-7.

### Detailed Description of the Invention

### (Vector)

The vector includes a nucleic acid sequence encoding protelomerase, a pair of nucleic acid sequences recognized by the protelomerase, and a nucleic acid sequence encoding a protein. The vector may further include other sequences.

The vector can be used as a vector for production of a linear covalently closed DNA.

In the present invention, the "linear covalently closed DNA" is a linear double-stranded DNA having a structure where ends thereof are each closed with a hairpin structure, and is abbreviated as LCC DNA.

In the present invention, the "vector" encompasses an artificially constructed nucleic acid molecule.

In the present invention, the "nucleic acid" may be also referred to as a "polynucleotide," and is a DNA or RNA, preferably a DNA. The DNA may be a single-stranded DNA or double-stranded DNA.

A structure of the vector is not particularly limited, and may be appropriately selected according to the intended purpose. Examples of the structure of the vector include a circular plasmid vector, a linear DNA vector, an artificial chromosome, and the like. Among the above-listed examples, a circular plasmid vector is preferred, and a double-stranded circular plasmid DNA vector is more preferred.

In the present invention, the "double-stranded circular plasmid DNA" encompasses a cyclic double-stranded DNA that is replicated within a cell.

### <Nucleic acid sequence encoding protelomerase>

In the present invention, the "protelomerase" encompasses a polypeptide capable of cleaving certain palindromic sequences (nucleic acid sequences recognized by protelomerase) and rebinding to produce a linear covalently closed DNA.

The protelomerase is not particularly limited as long as the protelomerase is a polypeptide having the above-described function. Examples of the protelomerase include Agrobacterium fabrum-derived protelomerase (TelA protelomerase, ACCESSION No. AAK88254), Halomonas virus HAP1-derived protelomerase (ACCESSION No. ABY90402), Vibrio virus VP882-derived protelomerase (ACCESSION No. ABM73418), Klebsiella phage phiKO2-derived protelomerase (TelK protelomerase, ACCESSION No. AAR83042), Rhizobium pusense-derived protelomerase (ACCESSION No. QKJ91773), Feldmannia species virus-derived protelomerase (ACCESSION No. ACH46812), Vibrio phage vB_VpaM_MAR-derived protelomerase (ACCESSION No. AFV81380), Yersinia phage PY54-derived protelomerase (Tel protelomerase, ACCESSION No. CAD91792), Escherichia virus N15-derived protelomerase (TelN protelomerase, ACCESSION No. AAB81106), and variants of any of the above-listed examples of the protelomerase.

In the present invention, moreover, the protelomerase also includes protelomerase that was originally protelomerase but has lost the function of the protelomerase due to artificial modification or mutation.

Among the above-listed examples, TelA protelomerase, TelK protelomerase, Tel protelomerase, TelN protelomerase, or a variant thereof is preferred, TelN protelomerase or a variant thereof is more preferred.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original amino acid sequence.

In the present invention, the "polypeptide" encompasses a molecule in which two or more amino acids are joined by peptide bonds, and includes, other than proteins, short-chain molecules, such as peptides and oligopeptides.

In the present invention, the "nucleic acid sequence encoding protelomerase" encompasses a nucleic acid sequence designed, based on the codon table, for a polypeptide composed of an amino acid sequence constituting protelomerase. Transcription and translation of the nucleic acid sequence lead to generation of the polypeptide.

The nucleic acid sequence encoding protelomerase is not particularly limited, and may be appropriately selected according to the intended purpose. The nucleic acid sequence encoding protelomerase is preferably a nucleic acid sequence encoding TelA protelomerase, TelK protelomerase, Tel protelomerase, TelN protelomerase, or a variant thereof, more preferably a nucleic acid sequence encoding TelN protelomerase or a variant thereof, and yet more preferably a nucleic acid sequence of SEQ ID NO: 1 or a variant thereof.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original nucleic acid sequence.

### <Pair of nucleic acid sequences recognized by protelomerase>

In the present invention, the "pair of nucleic acid sequences recognized by protelomerase" encompasses a pair of nucleic acid sequences, which are recognized by the protelomerase to cleave and rebind to produce a linear covalently closed DNA.

Examples of the nucleic acid sequences recognized by protelomerase include certain palindromic sequences. The nucleic acid sequences recognized by protelomerase are not particularly limited, and may be appropriately selected according to the intended purpose. Nucleic acid sequences recognized by TelA protelomerase, nucleic acid sequences recognized by TelK protelomerase, nucleic acid sequences recognized by Tel protelomerase, or nucleic acid sequences recognized by TelN protelomerase are preferred, nucleic acid sequences recognized by TelN protelomerase are more preferred, and nucleic acid sequences of SEQ ID NO: 2 (telRL sequence) or variants thereof are yet more preferred.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original nucleic acid sequence.

### <Nucleic acid sequence encoding protein>

The nucleic acid sequence encoding a protein is a nucleic acid sequence located between the pair of nucleic acid sequences recognized by protelomerase.

Specifically, the nucleic acid sequence recognized by protelomerase is present at an anterior site of the nucleic acid sequence encoding a protein, and the nucleic acid sequence recognized by protelomerase is present at a posterior site of the nucleic acid sequence encoding a protein.

In the present invention, the "nucleic acid sequence (gene) encoding a protein" encompasses not only DNA, but also mRNA and cDNA, among nucleic acids included in host cells or viruses. Typically, the "nucleic acid sequence (gene) encoding a protein" may be DNA, particularly genomic DNA.

The "nucleic acid sequence (gene) encoding a protein" is not restricted in terms of a functional region thereof. For example, the nucleic acid sequence (gene) may include only exons, or may include exons and introns. In an embodiment where the "nucleic acid sequence (gene) encoding a protein" is RNA, any one or more, or all of bases of thymine (T) in the nucleic acid sequence may be read as uracil (U).

In the present invention, the "nucleic acid sequence (gene) encoding a protein" encompasses a gene encoding a polypeptide a cell produces, where the polypeptide may be an endogenous polypeptide or heterologous polypeptide for the cell.

The nucleic acid sequence (gene) encoding a protein is not particularly limited, and may be appropriately selected according to the intended purpose. The nucleic acid sequence (gene) may be a target gene introduced into a vector.

The polypeptide encoded by the target gene that is introduced into the vector is not particularly limited, and may be appropriately selected according to the intended purpose. Examples of the polypeptide encoded by the target gene include: polypeptides constituting viruses; polypeptides produced by animals, plants, fungi, algae, bacteria, viruses, and the like; partial peptide fragments of the foregoing polypeptides; and the like. The above-listed examples may be used as a cellular or gene therapy agent, vaccine, a therapeutic agent, or the like.

The polypeptides constituting viruses are not particularly limited, and may be appropriately selected according to the intended purpose. Examples of the polypeptides constituting viruses include polypeptides constituting parvovirus, polypeptides constituting adeno-associated virus (AAV), polypeptides constituting adenovirus, polypeptides constituting coronavirus, polypeptides constituting retrovirus, polypeptides constituting lentivirus, polypeptides constituting herpesvirus, polypeptides constituting poliovirus, polypeptides constituting papillomavirus, polypeptides constituting vaccinia virus, polypeptides constituting poxvirus, and the like.

The polypeptides constituting adeno-associated virus (AAV) are not particularly limited, and may be appropriately selected according to the intended purpose. Examples of the polypeptides constituting adeno-associated virus (AAV) include adeno-associated virus-derived VP1, adeno-associated virus-derived VP2, adeno-associated virus-derived VP3, adeno-associated virus-derived Rep (Rep protein), adeno-associated virus-derived Cap (Capsid protein), adeno-associated virus-derived AAP, adeno-associated virus-derived MAAP, and the like.

The polypeptides constituting adenovirus are not particularly limited, and may be appropriately selected according to the intended purpose. Examples of the polypeptides include adenovirus-derived E2A, adenovirus-derived E4, adenovirus-derived VA, adenovirus-derived E1A, adenovirus-derived E1B, and the like.

The polypeptides constituting coronavirus are not particularly limited, and may be appropriately selected according to the intended purpose. Examples of the polypeptides constituting coronavirus include spike polypeptides of coronavirus, nucleocapsid polypeptides of coronavirus, membrane polypeptides of coronavirus, envelope polypeptides of coronavirus, and the like.

The polypeptides produced by the animals, plants, fungi, algae, bacteria, viruses, or the like are not particularly limited, and may be appropriately selected according to the intended purpose. Examples of the polypeptide include enzymes (e.g., such as phytase, amylase, glucosidase, cellulase, lipase, protease, glutaminase, peptidase, nuclease, oxidase, lactase, xylanase, trypsin, pectinase, isomerase, and the like), antibody-binding proteins (e.g., protein A, protein G, protein L, and the like), antibodies or antibody fragments (e.g., human antibodies, humanized antibodies, chimeric antibodies, llama antibodies, alpaca antibodies, single-chain antibodies, heavy-chain antibodies, multivalent antibodies, Fab, F(ab'), F(ab')₂, Fc, Fc-fusion protein, bispecific antibodies, heavy chain (H-chain), light chain (L-chain), single chain Fv (scFv), sc (Fv)₂, disulfide-linked Fv (sdFv), Diabodies, antibody-like molecular target peptide (micro antibody)), or complexes (antibody-like molecules) of compounds other than the antibodies or antibody fragments (e.g., other functional proteins) and an antibody fragment, serum albumin, such as human serum albumin, and the like; epidermal growth factors, such as human epidermal growth factors; insulin, growth hormone, erythropoietin, interferon, blood-coagulation factor VIII, granulocyte-colony stimulating factor (G-CSF), granulocyte-microphage colony-stimulating factor (GM-CSF), thrombopoietin, IL-1, IL-6, tissue plasminogen activator (TPA), urokinase, leptin, stem cell growth factor (SCF), fibroin, fluorescent protein, Hepatitis B virus surface antigen, hirudin, and the like.

As described above, the vector of the present invention includes a nucleic acid sequence encoding protelomerase, a pair of nucleic acid sequences recognized by the protelomerase, and a nucleic acid sequence encoding a protein and placed between the pair of the nucleic acid sequences. A type of each nucleic acid sequence used is not particularly limited, and the vector may include one, two, three or more types of sequences.

### <Other sequences>

The above-mentioned other sequences are not particularly limited, and may be appropriately selected according to the intended purpose. Examples of other sequences include a nucleic acid sequence encoding an activator or repressor protein (e.g., a LacI gene, an AraC gene, and the like), a nucleic acid sequence regulating expression of the protelomerase, a cloning site including one or more restriction enzyme recognition sites, an overlapping region for using the In-Fusion Cloning System of Clontech Laboratories, Inc., Gibson Assembly System of New England Biolabs, Inc. or the like, a nucleic acid sequence of a selectable marker gene (e.g., a nucleic acid sequence encoding an antibiotic resistant protein), and the like. The sequences, such as nucleic acid sequences of the cloning site, the overlapping region, and the selectable marker gene may be nucleic acid molecules in the form where any of the foregoing sequences can be added (e.g., a nucleic acid molecule having a sequence including one or more restriction enzyme recognition sites to which any of the foregoing sequences can be added).

The LacI gene sequence is not particularly limited, and may be appropriately selected according to the intended purpose. The LacI gene sequence is preferably a nucleic acid sequence of SEQ ID NO: 23 or a variant thereof.

The AraC gene sequence is not particularly limited, and may be appropriately selected according to the intended purpose. The AraC gene sequence is a nucleic acid sequence of SEQ ID NO: 3 or a variant thereof.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original nucleic acid sequence.

In the present invention, the "nucleic acid sequence regulating expression" may be also referred to as a "promoter," and encompasses a nucleic acid sequence region located upstream of the nucleic acid sequence encoding the polypeptide. In addition to RNA polymerase, various transcription modulators associated with acceleration or repression of transcription are linked to or acted on the nucleic acid sequence region, and as a result, the nucleic acid sequence encoding the polypeptide serving as a template is read to synthesize (transcribe) a complementary RNA.

The nucleic acid sequence regulating expression is not particularly limited, provided that the nucleic acid sequence is a nucleic acid sequence for inducing expression in the selected conditions. The nucleic acid sequence regulating expression may be appropriately selected according to the intended purpose. Examples of the nucleic acid sequence regulating expression include heat-inducible promoters (lambda PR, lambda PL, etc.), arabinose-inducible promoters (araBAD promoters, etc.), IPTG-inducible promoters (LAC promoters, LACUV5 promoters, TAC promoters, Trc promoters, LPP promoters, etc.), T7 promoters, cold (low temperature)-inducible promoters (cspA promoters, etc.), Trp promoters, rhamnose-inducible promoters (rhaT promoter, etc.), proU promoters, prpB promoters, phoA promoters, recA promoters, tetA promoters, cadA promoters, variants of the foregoing promoters, and the like.

A method of acquiring the variant is not particularly limited, and may be appropriately selected according to the intended purpose. Examples of the method include: a method of substituting, inserting, adding, or deleting a nucleic acid sequence to which an activator or repressor is linked; a method of substituting, inserting, adding, or deleting a nucleic acid sequence to which a transcription factor is linked; a method of substituting, inserting, adding or deleting a nucleic acid sequence to which RNA polymerase is linked; a method of substituting, inserting, adding, or deleting a nucleic acid sequence to which a ribosome is linked; a method of substituting, inserting, adding, or deleting a region of a nucleic acid sequence, which is from a transcription start site to a start codon, to improve or lower stability of mRNA; a method of preparing a library of the nucleic acid sequences to perform screening; a method of linking and fusing a plurality of promoters; and the like.

Among the above-listed examples, an IPTG-inducible promoter or arabinose-inducible promoter is preferred, an arabinose-inducible promoter is more preferred, and a nucleic acid sequence of SEQ ID NO: 4 or a variant thereof is yet more preferred.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original nucleic acid sequence.

### <Vector production method>

The vector production method is not particularly limited, and may be appropriately selected according to the intended purpose. Examples of the vector production method include total synthesis, PCR, and a method of using the In-Fusion Cloning System of Clontech Laboratories, Inc., Gibson Assembly System of New England Biolabs, Inc., or the like.

### (Linear covalently closed DNA production method)

The linear covalently closed DNA production method includes a gene introduction step, and may further include other steps A.

### <Gene introduction step>

The gene introduction step is a step that includes introducing, into a host, a vector including a nucleic acid sequence encoding protelomerase, a pair of nucleic acid sequences recognized by the protelomerase, and a nucleic acid sequence located between the pair of the nucleic acid sequences and encoding a protein.

The vector including the nucleic acid sequence encoding protelomerase, the pair of the nucleic acid sequences recognized by protelomerase, and the nucleic acid sequence located between the pair of nucleic acid sequences and encoding a protein is as described above.

The host is not particularly limited, and may be appropriately selected according to the intended purpose. Examples of the host include: archaea; eubacteria, such as *E. coli,* lactic acid bacteria, *Bacillus subtilis,* and the like; eukaryotes, such as animal cells, plant cells, insect cells, mold, yeast cells, and the like; viruses; and the like. Among the above-listed examples, eubacteria are preferred, and *E. coli* is more preferred.

The *E. coli* is not particularly limited, and may be appropriately selected according to the intended purpose. As the *E. coli, Escherichia* genus is preferred, *Escherichia coli* is more preferred, and *Escherichia coli* strain K-12, *Escherichia coli* strain B, and the like are yet more preferred. Moreover, commercially available *E. coli* strains or *E. coli* strains available from the bioresource centers may be used.

The commercially available *E. coli* strains or *E. coli* strains available from the bioresource centers are not particularly limited, and may be appropriately selected according to the intended purpose. Examples of the commercially available *E. coli* strains or *E. coli* strains available from the bioresource centers include the strain JM109, the strain DH5, the strain DH5α, the strain DH10B, the strain NEB10β, the strain HST08, the strain HST16CR, the strain HB101, the strain W3110, the strain MG1655, the strain BL21, the strain BL21 DE3, and the like. The above-listed strains can be obtained from New England Biolabs, Inc., TAKARA BIO INC., Thermo Fisher Scientific Inc., TOYOBO CO., LTD., American Type Culture Collection (ATCC), National Bio Resource Project (NBRP), and the like.

In the present invention, moreover, strains derived from the above-listed *E. coli* strains may be used. Examples of the derived strains include the strain JW3973 that is methionine-auxotrophic (available from NBRP), the strain JW2806 that is leucine-auxotrophic (available from NBRP), the strain JW3582 that is cysteine-auxotrophic (available from NBRP), the strain ME5305 that is thiamine and histidine-auxotrophic (available from NBRP), and the like.

In the present invention, the "gene introduction step" encompasses a step that includes transforming a host with the predetermined vector. By transforming a host with the vector of the present invention, a linear covalently closed DNA can be produced.

In the present invention, the "transformation" encompasses introduction of a vector into a host.

As a method of introducing the vector into a host, i.e., a transformation method, any method known in the related art is suitably used. In the case where *E. coli* is used as a host, examples of the method include, but are not limited to, the heat shock method, electroporation, and the like.

In Examples described later, a vector including a nucleic acid sequence (SEQ ID NO: 1) encoding protelomerase, a pair of nucleic acid sequences (SEQ ID NO: 2) recognized by the protelomerase, and a nucleic acid sequence (SEQ ID NO: 5) located between the pair of nucleic acid sequences and encoding a target protein was prepared, and *E. coli* strain NEB10β was transformed with the vector to produce a linear covalently closed DNA.

### <Other steps A>

Other steps A are not particularly limited, and may be appropriately selected according to the intended purpose. Examples of the other steps A include an expression induction step of inducing expression of the protelomerase after the gene introduction step, a purification step of a linear covalently closed DNA, and the like.

The expression induction step of inducing expression of the protelomerase after the gene introduction step is not particularly limited, and may be appropriately selected according to the intended purpose. Examples of the expression induction step include a method of adding an expression inducer to a fluid culture medium of a gene-introduced host, and the like.

In the case where an arabinose-inducible promoter is used as the nucleic acid sequence regulating expression of the protelomerase, arabinose can be used as the expression inducer.

In the case where an IPTG-inducible promoter is used as the nucleic acid sequence regulating expression of the protelomerase, IPTG is used as the expression inducer.

The purification step of the linear covalently closed DNA is not particularly limited, and may be appropriately selected according to the intended purpose. Examples of the purification step include: a method of purifying using a commercially available kit; a method of purifying a cell solution by cation exchange chromatography, anion exchange chromatography, or size exclusion chromatography, or using a filter or ultrafiltration membrane; and the like.

### (Parvovirus vector production method)

The parvovirus vector production method includes a gene introduction step and a transfection step, and may further include other steps B.

### <Parvovirus>

In the present invention, the "parvovirus" encompasses a virus belongs to the family *Parvoviridae,* and is a virus including a linear single-stranded DNA inside a capsid, and forming a regular icosahedral structure.

A type of the parvovirus is not particularly limited, and may be appropriately selected according to the intended purpose. Examples of the parvovirus include canine parvovirus, feline parvovirus, bovine parvovirus, parvovirus B19, bocavirus, densovirus, adeno-associated virus (AAV), and the like. Among the above-listed examples, adeno-associated virus (AAV) is preferable.

The adeno-associated virus (AAV) is not particularly limited, and may be appropriately selected according to the intended purpose. Examples of the adeno-associated virus (AAV) include AAV serotype 1 (AAV1), AAV serotype 2 (AAV2), AAV serotype 3 (AAV3), AAV serotype 4 (AAV4), AAV serotype 5 (AAV5), AAV serotype 6 (AAV6), AAV serotype 7 (AAV7), AAV serotype 8 (AAV8), AAV serotype 9 (AAV9), AAV serotype 10 (AAV10), AAV serotype 11 (AAV11), AAV serotype 12 (AAV12), AAV serotype 13 (AAV13), AAV serotype 14 (AAV14), AAV serotype rh10 (AAVrh10), variants of the foregoing AAVs, and the like. Among the above-listed examples, AAV serotype 2 (AAV2), AAV serotype 5 (AAV5), AAV serotype 8 (AAV8), AAV serotype 9 (AAV9), or a variant of any of the foregoing AAVs is preferred; AAV serotype 2 (AAV2), AAV serotype 9 (AAV9), or a variant of AAV2 or AAV9 is more preferred.

The variant of the adeno-associated virus is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original amino acid sequence.

Amino acids constituting the parvovirus may be natural amino acids, unnatural amino acids, or modified amino acids.

Moreover, an amino acid sequence of the parvovirus may be an amino acid sequence of de-novo design.

### <Gene introduction step>

The gene introduction step is a step that includes introducing, into a first host, a vector including a nucleic acid sequence encoding protelomerase, a pair of nucleic acid sequences recognized by the protelomerase, and a nucleic acid sequence located between the pair of nucleic acid sequences and encoding a protein.

In the present invention, the "gene introduction step" encompasses a step that includes transforming a first host with the predetermined vector. By transforming a first host with the vector of the present invention, a linear covalently closed DNA is produced.

In the present invention, "transformation" encompasses introduction of the vector into a first host.

As a method of introducing the vector into a first host, i.e., a transformation method, any method known in the related art is suitably used. In the case where *E. coli* is used as the first host, examples of the method include, but are not limited to, the heat shock method, electroporation, and the like.

### <<Vector>>

The vector is as described above. The vector includes a nucleic acid sequence encoding protelomerase, a pair of nucleic acid sequences recognized by the protelomerase, and a nucleic acid sequence encoding a protein. The vector may further include other sequences.

The vector can be used as a vector for production of a linear covalently closed DNA.

In the present invention, "linear covalently closed DNA" encompasses a linear double-stranded DNA having a structure where each end is closed with a hairpin structure, and is abbreviated as LCC DNA.

In the present invention, the "vector" encompasses an artificially constructed nucleic acid molecule.

In the present invention, the "nucleic acid" may be referred to as a "polynucleotide," encompasses DNA or RNA, and is preferably DNA. The DNA may be a single-stranded DNA or a double-stranded DNA.

The structure of the vector is not particularly limited, and may be appropriately selected according to the intended purpose. Examples of the structure of the vector include a circular plasmid vector, a linear DNA vector, an artificial chromosome, and the like. Among the above-listed examples, a circular plasmid vector is preferred, and a double-stranded circular plasmid DNA vector is more preferred.

In the present invention, the "double-stranded circular plasmid DNA" encompasses a circular double-stranded DNA plasmid replicated within a cell.

<<<Nucleic acid sequence encoding protelomerase>>>

As described above, in the present invention, the "protelomerase" encompasses a polypeptide capable of cleaving and rebinding specific palindromic sequences (nucleic acid sequences recognized by protelomerase) to produce a linear covalently closed DNA.

The protelomerase is not particularly limited, provided that the protelomerase has the above-described functions. Examples of the protelomerase include Agrobacterium fabrum-derived protelomerase (TelA protelomerase, ACCESSION No. AAK88254), Halomonas virus HAP1-derived protelomerase (ACCESSION No. ABY90402), Vibrio virus VP882-derived protelomerase (ACCESSION No. ABM73418), Klebsiella phage phiKO2-derived protelomerase (TelK protelomerase, ACCESSION No. AAR83042), Rhizobium pusense-derived protelomerase (ACCESSION No. QKJ91773), Feldmannia species virus-derived protelomerase (ACCESSION No. ACH46812), Vibrio phage vB_VpaM_MAR-derived protelomerase (ACCESSION No. AFV81380), Yersinia phage PY54-derived protelomerase (Tel protelomerase, ACCESSION No. CAD91792), Escherichia virus N15-derived protelomerase (TelN protelomerase, ACCESSION No. AAB81106), variants thereof, and the like.

Moreover, protelomerase that was originally protelomerase but has lost the functions thereof due to artificial modification or mutation is also included in the protelomerase associated with the present invention.

Among the above-listed examples, TelA protelomerase, TelK protelomerase, Tel protelomerase, TelN protelomerase, or a variant thereof is preferable, and TelN protelomerase or a variant thereof is more preferable.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original amino acid sequence.

In the present invention, the "polypeptide" encompasses a polypeptide in which two or more amino acids are linked with peptide bonds, and includes, other than proteins, short-chain molecules, such as peptides and oligopeptides.

In the present invention, the "nucleic acid sequence encoding protelomerase" encompasses a nucleic acid sequence designed, based on the codon table, for a polypeptide composed of an amino acid sequence constituting protelomerase. Transcription and translation of the nucleic acid sequence lead to generation of the polypeptide.

The nucleic acid sequence encoding protelomerase is not particularly limited, and may be appropriately selected according to the intended purpose. The nucleic acid sequence encoding protelomerase is preferably a nucleic acid sequence encoding TelA protelomerase, TelK protelomerase, Tel protelomerase, TelN protelomerase, or a variant thereof, more preferably a nucleic acid sequence encoding TelN protelomerase or a variant thereof, and yet more preferably a nucleic acid sequence of SEQ ID NO: 1 or a variant thereof.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original nucleic acid sequence.

### <<<Pair of nucleic acid sequences recognized by protelomerase>>>

As described above, in the present invention, the "pair of nucleic acid sequences recognized by protelomerase" encompasses a pair of nucleic acid sequences, which are recognized by the protelomerase, the sites of which are cleaved, and the ends of which are rebound to produce a linear covalently closed DNA.

Examples of the nucleic acid sequences recognized by the protelomerase includes certain palindromic sequences. The nucleic acid sequences recognized by the protelomerase are not particularly limited, and may be appropriately selected according to the intended purpose. Nucleic acid sequences recognized by TelA protelomerase, nucleic acid sequences recognized by TelK protelomerase, nucleic acid sequences recognized by Tel protelomerase, or nucleic acid sequences recognized by TelN protelomerase are preferred, nucleic acid sequences recognized by TelN protelomerase are more preferred, and nucleic acid sequences of SEQ ID NO: 2 (telRL sequence) or variants thereof are yet more preferred.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original nucleic acid sequence.

### <<<Nucleic acid sequence encoding protein>>>

The nucleic acid sequence encoding a protein is a nucleic acid sequence located between the pair of nucleic acid sequences recognized by protelomerase.

Specifically, the nucleic acid sequence recognized by protelomerase is present at an anterior site of the nucleic acid sequence encoding a protein, and the nucleic acid sequence recognized by protelomerase is present at a posterior site of the nucleic acid sequence encoding a protein.

In the present invention, the "nucleic acid sequence (gene) encoding a protein" encompasses not only DNA, but also mRNA and cDNA, among nucleic acids which host cells or viruses have. Typically, the "nucleic acid sequence (gene) encoding a protein" may be DNA, particularly genomic DNA.

The "nucleic acid sequence (gene) encoding a protein" is not restricted in terms of a functional region thereof. For example, the nucleic acid sequence (gene) may include only exons, or may include exons and introns. In an embodiment where the "nucleic acid sequence (gene) encoding a protein" is RNA, any one or more, or all of bases of thymine (T) in the nucleic acid sequence may be read as uracil (U).

The "nucleic acid sequence (gene) encoding a protein" encompasses a gene encoding a polypeptide a cell produces, where the polypeptide may be an endogenous polypeptide, or heterologous polypeptide for the cell.

The nucleic acid sequence (gene) encoding a protein is not particularly limited, and may be appropriately selected according to the intended purpose. The nucleic acid sequence (gene) encoding a protein is preferably a target gene that is introduced into a parvovirus vector (a nucleic acid sequence encoding a target protein), a packaging gene (e.g., Rep (Rep protein) gene, Cap (Capsid protein) gene, and the like), or a helper gene (e.g., an adenovirus helper gene and the like). The above-listed genes may be used in combination.

The polypeptide encoded by the target gene introduced into the parvovirus vector (a target protein) is not particularly limited, and may be appropriately selected according to the intended purpose. Examples of the polypeptide encoded by the target gene introduced into the parvovirus vector include: polypeptides produced by animals, plants, fungi, algae, bacteria, and viruses; partial peptide fragments of the foregoing polypeptides; and the like. The above-listed examples may be used as a cellular or gene therapy agent, vaccine, a therapeutic agent, or the like.

The polypeptides produced by animals, plants, fungi, algae, bacteria, viruses or the like are is not particularly limited, and may be appropriately selected according to the intended purpose. Examples of the polypeptides produced by animals, plants, fungi, algae, bacteria, viruses or the like include enzymes (e.g., such as phytase, amylase, glucosidase, cellulase, lipase, protease, glutaminase, peptidase, nuclease, oxidase, lactase, xylanase, trypsin, pectinase, isomerase, and the like), antibody-binding proteins (e.g., protein A, protein G, protein L, and the like), antibodies or antibody fragments (e.g., human antibodies, humanized antibodies, chimeric antibodies, llama antibodies, alpaca antibodies, single-chain antibodies, heavy-chain antibodies, multivalent antibodies, Fab, F(ab'), F(ab')₂, Fc, Fc-fusion protein, bispecific antibodies, heavy chain (H-chain), light chain (L-chain), single chain Fv (scFv), sc (Fv)₂, disulfide-linked Fv (sdFv), Diabodies, antibody-like molecular target peptide (micro antibody)), or complexes (antibody-like molecules) of compounds other than the antibodies or antibody fragments (e.g., other functional proteins) and an antibody fragment, serum albumin, such as human serum albumin, and the like; epidermal growth factors, such as human epidermal growth factors; insulin, growth hormone, erythropoietin, interferon, blood-coagulation factor VIII, granulocyte-colony stimulating factor (G-CSF), granulocyte-microphage colony-stimulating factor (GM-CSF), thrombopoietin, IL-1, IL-6, tissue plasminogen activator (TPA), urokinase, leptin, stem cell growth factor (SCF), fibroin, fluorescent protein, Hepatitis B virus surface antigen, hirudin, and the like.

The polypeptide encoded by the packaging gene (packaging protein) is not particularly limited, and may be appropriately selected according to the intended purpose. Examples of the packaging protein include Rep (Rep protein), Cap (Capsid protein), and the like. Among the above-listed examples, a polypeptide constituting parvovirus (e.g., parvovirus-derived Rep, parvovirus-derived Cap, etc.) is preferable.

The polypeptide constituting parvovirus is not particularly limited, and may be appropriately selected according to the intended purpose. Examples of the polypeptide constituting parvovirus include a polypeptide constituting canine parvovirus, a polypeptide constituting feline parvovirus, a polypeptide constituting bovine parvovirus, a polypeptide constituting parvovirus B19, a polypeptide constituting bocavirus, a polypeptide constituting densovirus, a polypeptide constituting adeno-associated virus (AAV), and the like. A polypeptide constituting adeno-associated virus (AAV) is preferred.

The polypeptide constituting adeno-associated virus (AAV) is not particularly limited, and may be appropriately selected according to the intended purpose. Examples of the polypeptide constituting adeno-associated virus (AAV) include adeno-associated virus-derived VP1, adeno-associated virus-derived VP2, adeno-associated virus-derived VP3, adeno-associated virus-derived Rep, adeno-associated virus-derived Cap, adeno-associated virus-derived AAP, adeno-associated virus-derived MAAP, and the like.

Among the above-listed examples, adeno-associated virus-derived Rep or adeno-associated virus-derived Cap is preferred, adeno-associated virus-derived Rep and adeno-associated virus-derived Cap are more preferred.

The polypeptide encoded by the helper gene (helper protein) is not particularly limited, and may be appropriately selected according to the intended purpose. Examples of the polypeptide encoded by the helper gene (helper protein) include an adenovirus helper protein, and the like. The adenovirus helper protein is not particularly limited, and may be appropriately selected according to the intended purpose. Examples of the adenovirus helper protein include adenovirus-derived E2A, adenovirus-derived E4, adenovirus-derived VA, adenovirus-derived E1A, adenovirus-derived E1B, and the like.

Among the above-listed examples, adenovirus-derived E2A, adenovirus-derived E4, or adenovirus-derived VA is preferred, and adenovirus-derived E2A, adenovirus-derived E4, and adenovirus-derived VA are more preferred.

As described above, the vector of the present invention includes a nucleic acid sequence encoding protelomerase, a pair of nucleic acid sequences recognized by the protelomerase, and a nucleic acid sequence located between the pair of nucleic acid sequences and encoding a protein. A type of each nucleic acid sequence used is not particularly limited, and the vector may include one, two, three or more types of sequences.

### <<<Other sequences>>>

The above-mentioned other sequences are not particularly limited, and may be appropriately selected according to the intended purpose. Examples of other sequences include a nucleic acid sequence encoding an activator or repressor protein (e.g., a LacI gene, an AraC gene, etc.), a nucleic acid sequence regulating expression of the protelomerase, a cloning site including one or more restriction enzyme recognition sites, an overlapping region for using In-Fusion Cloning System of Clontech Laboratories, Inc., Gibson Assembly System of New England Biolabs, Inc. or the like, a nucleic acid sequence of a selectable marker gene (e.g., a nucleic acid sequence encoding an antibiotic resistant protein, such as AmpR and the like), a replication factor sequence of a host, and the like. The sequences, such as nucleic acid sequences of the cloning site, the overlapping region, and the selectable marker gene, may be nucleic acid molecules in the form where any of the foregoing sequences can be added (e.g., a nucleic acid molecule having a sequence including one or more restriction enzyme recognition sites to which any of the foregoing sequences can be added).

The LacI gene sequence is not particularly limited, and may be appropriately selected according to the intended purpose. The LacI gene sequence is preferably a nucleic acid sequence of SEQ ID NO: 23 or a variant thereof.

The AraC gene sequence is not particularly limited, and may be appropriately selected according to the intended purpose. The AraC gene sequence is preferably a nucleic acid sequence of SEQ ID NO: 3 or a variant thereof.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original nucleic acid sequence.

In the present invention, the "nucleic acid sequence regulating expression" may be also referred to as a "promoter," and encompasses a nucleic acid sequence region located upstream of the nucleic acid sequence encoding the polypeptide. In addition to RNA polymerase, various transcription modulators associated with acceleration or repression of transcription are linked to or acted on the nucleic acid sequence region, and as a result, the nucleic acid sequence encoding the polypeptide serving as a template is read to synthesize (transcribe) a complementary RNA.

The nucleic acid sequence regulating expression is not particularly limited, provided that the nucleic acid sequence is a nucleic acid sequence for inducing expression in the selected conditions. The nucleic acid sequence regulating expression may be appropriately selected according to the intended purpose. Examples of the nucleic acid sequence regulating expression include heat-inducible promoters (lambda PR, lambda PL, etc.), arabinose-inducible promoters (araBAD promoters, etc.), IPTG-inducible promoters (LAC promoters, LACUV5 promoters, TAC promoters, Trc promoters, LPP promoters, etc.), T7 promoters, cold (low temperature)-inducible promoters (cspA promoters, etc.), Trp promoters, rhamnose-inducible promoters (rhaT promoters, etc.), proU promoters, prpB promoters, phoA promoters, recA promoters, tetA promoters, cadA promoters, variants of the foregoing promoters, and the like.

A method of acquiring the variant is not particularly limited, and may be appropriately selected according to the intended purpose. Examples of the method include: a method of substituting, inserting, adding, or deleting a nucleic acid sequence to which an activator or repressor is linked; a method of substituting, inserting, adding, or deleting a nucleic acid sequence to which a transcription factor is linked; a method of substituting, inserting, adding or deleting a nucleic acid sequence to which RNA polymerase is linked; a method of substituting, inserting, adding, or deleting a nucleic acid sequence to which a ribosome is linked; a method of substituting, inserting, adding, or deleting a region of a nucleic acid sequence, which is from a transcription start site to a start codon, to improve or lower stability of mRNA; a method of preparing a library of the nucleic acid sequences to perform screening; a method of linking and fusing a plurality of promoters; and the like.

Among the above-listed examples, an IPTG-inducible promoter and an arabinose-inducible promoter are preferred, an arabinose-inducible promoter is more preferred, a nucleic acid sequence of SEQ ID NO: 4 or a variant thereof is yet more preferred.

The variant is not particularly limited, and may be appropriately selected according to the intended purpose. The variant preferably has 80% or greater sequence identity, more preferably 90% or greater sequence identity, yet more preferably 95% or greater sequence identity, and particularly preferably 99% or greater sequence identity of the original nucleic acid sequence.

### <<<Vector production method>>>

The vector production method is not particularly limited, and may be appropriately selected according to the intended purpose. Examples of the vector production method include total synthesis, PCR, and a method of using the In-Fusion Cloning System of Clontech Laboratories, Inc., Gibson Assembly System of New England Biolabs, Inc., or the like.

### <<First host>>

The first host is not particularly limited, and may be appropriately selected according to the intended purpose. Examples of the first host include: archaea; eubacteria, such as *E. coli,* lactic acid bacteria, *Bacillus subtilis,* and the like; eukaryotes, such as animal cells, plant cells, insect cells, mold, yeast cells, and the like; viruses; and the like. Among the above-listed examples, eubacteria are preferable, and *E*. *coli* is more preferable.

The *E. coli* is not particularly limited, and may be appropriately selected according to the intended purpose. As the *E. coli, Escherichia* genus is preferable, *Escherichia coli* is more preferable, and *Escherichia coli* strain K-12, *Escherichia coli* strain B, and the like are yet more preferable. Moreover, commercially available *E. coli* strains or *E. coli* strains available from the bioresource centers may be used.

The commercially available *E. coli* strains or *E. coli* strains available from the bioresource centers are not particularly limited, and may be appropriately selected according to the intended purpose. Examples of the commercially available *E. coli* strains or *E. coli* strains available from the bioresource centers include the strain JM109, the strain DH5, the strain DH5α, the strain DH10B, the strain NEB10β, the strain HST08, the strain HST16CR, the strain HB101, the strain W3110, the strain MG1655, the strain BL21, the strain BL21 DE3, and the like. The above-listed strains can be obtained from New England Biolabs, Inc., TAKARA BIO INC., Thermo Fisher Scientific Inc., TOYOBO CO., LTD., American Type Culture Collection (ATCC), National Bio Resource Project (NBRP), and the like.

In the present invention, moreover, strains derived from the above-listed *E. coli* strains may be used. Examples of the derived strains include the strain JW3973 that is methionine-auxotrophic (available from NBRP), the strain JW2806 that is leucine-auxotrophic (available from NBRP), the strain JW3582 that is cysteine-auxotrophic (available from NBRP), the strain ME5305 that is thiamine and histidine-auxotrophic (available from NBRP), and the like.

In Examples described later, a vector including a nucleic acid sequence (SEQ ID NO: 1) encoding protelomerase, a pair of nucleic acid sequences (SEQ ID NO: 2) recognized by the protelomerase, and a nucleic acid sequence (SEQ ID NO: 5) located between the pair of nucleic acid sequences and encoding a protein was prepared, and *E. coli* strain NEB10β was transformed with the vector to produce a linear covalently closed DNA.

In Examples described later, moreover, a vector including a nucleic acid sequence (SEQ ID NO: 1) encoding protelomerase, a pair of nucleic acid sequences (SEQ ID NO: 2) recognized by the protelomerase, and a nucleic acid sequence (Rep: SEQ ID NO: 12, Cap: SEQ ID NO: 13) located between the pair of nucleic acid sequences and encoding a packaging protein was prepared, and *E*. *coli* strain NEB10β was transformed with the vector to produce a linear covalently closed DNA.

In Examples described later, furthermore, a vector including a nucleic acid sequence (SEQ ID NO: 1) encoding protelomerase, a pair of nucleic acid sequences (SEQ ID NO: 2) recognized by the protelomerase, and a nucleic acid sequence (E2A: SEQ ID NO: 14, E4: SEQ ID NO: 15, VA: SEQ ID NO: 16) located between the pair of nucleic acid sequences and encoding a helper protein was prepared, and *E. coli* strain NEB10β was transformed with the vector to produce a linear covalently closed DNA.

In Examples described later, furthermore, a vector including a nucleic acid sequence (SEQ ID NO: 1) encoding protelomerase, a pair of nucleic acid sequences (SEQ ID NO: 2) recognized by the protelomerase, and a nucleic acid sequence located between the pair of nucleic acid sequences and encoding a packaging protein and a helper protein was prepared, and *E*. *coli strain* NEB10β was transformed with the vector to produce a linear covalently closed DNA.

### <Transfection step>

The transfection step is a step that includes transfecting a second host with the linear covalently closed DNA obtained in the gene introduction step.

### <<Second host>>

The second host is not particularly limited, and may be appropriately selected according to the intended purpose. Examples of the second host include: animal cells, such as HEK293 cells, HEK293T cells, Hela cells, CHO cells, iPS cells, mesenchymal stem cells, and the like; plant cells, such as cultured bamboo cells, cultured tobacco cells, and the like; insect cells, such as Sf9 cells and the like; yeast cells, such as *Saccharomyces cerevisiae* cells, *Pichia pastoris* cells, and the like; eubacteria, such as *E. coli,* and the like; and the like. Among the above-listed examples, animal cells or insect cells are preferable, and HEK293 cells, HEK293T cells, CHO cells, and Sf9 cells are more preferable.

Moreover, commercially available animal cells or animal cells available from the bioresource centers may be used.

The commercially available animal cells or animal cells available from the bioresource centers are not particularly limited, and may be appropriately selected according to the intended purpose. Examples of the commercially available animal cells or animal cells available from the bioresource centers include FreeStyle 293F, FreeStyle CHO-S, Expi293F, and the like.

The above-listed cells can be obtained from Thermo Fisher Scientific Inc., TOYOBO CO., LTD., TAKARA BIO INC., RIKEN, American Type Culture Collection (ATCC), and the like.

### <<Transfection>>

A method of transfecting the second host with the linear covalently closed DNA is not particularly limited, and may be appropriately selected according to the intended purpose. Examples of the method include a cationic lipid-mediated transfer method, calcium phosphate co-precipitation, a DEAE dextran-mediated transfer method, a cationic polymer-mediated transfer method, a virus-mediated transfer method, electroporation, a transfer method using nanoparticles, direct microinjection, a transfer method using laser, and the like. Among the above-listed examples, a transfer method mediated by a cationic polymer, such as polyethylene imine (PEI) is preferred.

A specific example of the parvovirus vector production method is not particularly limited, and may be appropriately selected according to the intended purpose. Examples of the parvovirus vector production method include: a parvovirus vector production method where, in the gene introduction step, a vector including the nucleic acid sequence encoding protelomerase, the pair of nucleic acid sequences recognized by the protelomerase, and the nucleic acid sequence located between the pair of the nucleic acid sequences and encoding a target protein is introduced into a first host, a vector including the nucleic acid sequence encoding protelomerase, the pair of nucleic acid sequences recognized by the protelomerase, and the nucleic acid sequence located between the pair of nucleic acid sequences and encoding a packaging protein is introduced into the first host, and a vector including the nucleic acid sequence encoding protelomerase, the pair of nucleic acid sequences recognized by the protelomerase, and the nucleic acid sequence located between the pair of nucleic acid sequences and encoding a helper protein is introduced into the first host, and in the transfection step, a second host is transfected with any of or all of the three linear covalently closed DNAs obtained in the gene introduction step, which are the linear covalently closed DNA including the nucleic acid sequence encoding a target protein (target gene), the linear covalently closed DNA including the nucleic acid sequence encoding a packaging protein (packaging gene), and the linear covalently closed DNA including the nucleic acid sequence encoding a helper protein (gene); a parvovirus vector production method where, in the gene introduction step, a vector including the nucleic acid sequence encoding protelomerase, the pair of nucleic acid sequences recognized by the protelomerase, and the nucleic acid sequence located between the pair of nucleic acid sequences and encoding a target protein is introduced into a first host, and a vector including a nucleic acid sequence encoding protelomerase, the pair of nucleic acid sequences recognized by the protelomerase, and the nucleic acid sequence located between the pair of nucleic acid sequences and encoding a packaging protein and a helper protein is introduced into the first host, and in the transfection step, a second host is transfected with either of or all of two linear covalently closed DNAs obtained in the gene introduction step, which are the linear covalently close DNA including the nucleic acid sequence encoding a target protein (target gene), and the linear covalently closed DNA including the nucleic acid sequence encoding a packaging protein and the nucleic acid sequence encoding a helper protein (packaging gene and helper gene); and the like.

In Example 2-8 described later, HEK293T cells were transfected with the obtained three linear covalently closed DNAs to produce an adeno-associated virus vector of the Parvoviridae family.

In Example 2-9 described later, HEK293T cells were transfected with the obtained two linear covalently closed DNAs to produce an adeno-associated virus vector of the Parvoviridae family.

According to the parvovirus vector production method, a production method of a high-quality parvovirus vector, which is highly efficient, and enables simple production using linear covalently closed DNAs substantially free from unnecessary sequences, can be provided.

The unnecessary sequences are not particularly limited, provided that the unnecessary sequences are sequences other than the nucleic acid sequences encoding proteins, i.e., nucleic acid sequences that are not intended to be inserted into a capsid of a parvovirus vector. Examples of the unnecessary sequences include a nucleic acid sequence encoding an antibiotic resistant protein (e.g., AmpR and the like), a replication factor sequence of a host, a packaging gene, a helper gene, a nucleic acid sequence encoding protelomerase, a nucleic acid sequence encoding an activator or repressor, a nucleic acid sequence regulating expression of protelomerase, and the like.

In the parvovirus vector production method, the number of vector genomes of sequences (unnecessary sequences) other than the nucleic acid sequence encoding a protein in the culture supernatant of the second host after the transfection step where the second host is transfected with the linear covalently closed DNA obtained in the gene introduction step is not particularly limited, and may be appropriately selected according to the intended purpose. The number of the vector genomes of the sequences (unnecessary sequences) is preferably 10% or less, more preferably 5% or less, yet more preferably 2.5% or less, and particularly preferably 1% or less, relative to the number of vector genomes of the nucleic acid sequence encoding a protein.

In the parvovirus vector production method, the number of vector genomes of sequences (unnecessary sequences) other than the nucleic acid sequence encoding a protein in the cell solution of the second host after the transfection step where the second host is transfected with the linear covalently closed DNA obtained in the gene introduction step is not particularly limited, and may be appropriately selected according to the intended purpose. The number of vector genomes of the sequences (unnecessary sequences) is preferably 1% or less, more preferably 0.5% or less, yet more preferably 0.1% or less, and particularly preferably 0.01% or less, relative to the number of the vector genomes of the nucleic acid sequence encoding a protein.

In the present invention, the "vector genome (vg)" encompasses a nucleic acid present inside a capsid of a virus. In the case of parvovirus, the vector genome (vg) encompasses a linear single-stranded DNA inside a capsid. As a method of measuring the quantity of viruses present within a sample, the number of the vector genomes can be represented as the physical titer of the virus.

The number of vector genomes of the sequences (unnecessary sequences) other than the nucleic acid sequence encoding a protein in the culture supernatant is measured by quantitative PCR in the following manner.

HEK293T cells (obtained from ATCC) are seeded on a 10 centimeter (cm) culture dish to be 6.3×10⁵ cells, followed by incubating in the atmosphere of 5% CO₂ at 37°C for 3 days. Three days later, transfection is carried out, followed by incubating in the atmosphere of 5% CO₂ at 37 degrees Celsius (°C) for 4 days. Four days later, the fluid culture medium of the culture dish is collected. To the culture dish, 3 milliliter (mL) of a PBS solution is added, and then the PBS solution is collected. To the culture dish, 1 mL of an Accutase solution (produced by Innovative Cell Technologies, Inc.) is added to separate the cells, and the resulting cell suspension liquid is collected. To the collected cell suspension liquid, a 6 mL of a PBS solution is added, and the resulting mixture is collected. The collected fluid is subjected to centrifugal separation to collect a supernatant. To the supernatant, 8 mL of a 2.5 M NaCl solution including 40% PEG8000 (produced by Sigma-Aldrich) is added, and the resulting mixture is left to stand while cooling on ice cubes for 2 hours. Centrifugal separation is carried out to remove a supernatant. To the resulting pellet, 1 mL of a PBS solution including 0.1% Triton X100 is added, and the resulting mixture is stirred at 4°C for 20 minutes. To the resulting mixture, 1 microliter (µL) of KANEKA Endonuclease (KEN 02500, produced by KANEKA CORPORATION, >250 U/µL) and 7.5 µL of a 1 M MgCl₂ solution are added, and the resulting mixture is left to stand at 37°C for 30 minutes. After the standing, 15 µL of a 0.5 M EDTA solution is added. Centrifugal separation is carried out, then the supernatant is collected to prepare a supernatant-derived sample (culture supernatant). Using the obtained supernatant-derived sample (culture supernatant), the number of vector genomes of the nucleic acid sequence encoding a protein, and the number of vector genomes of the gene encoding an antibiotic resistant protein, which is a sequence (unnecessary sequences) other than the nucleic acid sequence encoding protein, were measured by real-time PCR (Quantstudio, SYBR Green) that is one of the quantitative PCR methods, to calculate the number of the vector genomes of the sequences (unnecessary sequences) other than the nucleic acid sequence encoding a protein, relative to the number of the vector genomes of the nucleic acid sequence encoding a protein.

The number of vector genomes of the sequences (unnecessary sequences) other than the nucleic acid sequence encoding a protein in the cell solution can be measured by quantitative PCR in the following manner.

HEK293T cells (obtained from ATCC) are seeded on a 10 cm culture dish to be 6.3×10⁵ cells in the atmosphere of 5% CO₂ at 37°C for 3 days. Three days later, transfection is carried out, followed by incubating in the atmosphere of 5% CO₂ at 37°C for 4 days. Four days later, the fluid culture medium of the culture dish is removed. To the resulting culture, 3 mL of a PBS solution is added, followed by removing the PBS solution. To the culture, 1 mL of an Accutase solution (produced by Innovative Cell Technologies, Inc.) is added to separate the cells, to thereby collect a cell suspension liquid. To the resulting cell suspension liquid, 6 mL of a PBS solution is added, and the resulting mixture is collected. The collected liquid is subjected to centrifugal separation, to collect the cells. To the cells, 1 mL of a PBS solution including 0.1% Triton X100 is added, and the resulting mixture is stirred at 4°C for 20 minutes. To the resulting mixture, 1 µL of a solution of KANEKA Endonuclease (KEN02500, produced by KANEKA CORPORATION, >250U/pL) and 7.5 µL of a 1 M MgCl₂ solution are added, and the resulting mixture is left to stand at 37°C for 30 minutes. After the standing, 15 µL of a 0.5 M EDTA solution is added. Centrifugal separation is carried out to separate the supernatant, to thereby prepare a cell-derived sample (cell solution). Using the obtained cell-derived sample (cell solution), the number of vector genomes of the nucleic acid sequence encoding a protein, and the number of vector genomes of genes encoding antibiotic resistant proteins, which are sequences (unnecessary sequences) other than the nucleic acid sequence encoding a protein are measured by real-time PCR (Quantstudio, SYBR Green) that is one of the quantitative PCR methods, to calculate the number of the vector genomes of the sequences (unnecessary sequences) other than the nucleic acid sequence encoding a protein, relative to the number of vector genomes of the nucleic acid sequence encoding a protein.

As the gene encoding an antibiotic resistant protein, a gene encoding an antibiotic resistant protein included in the vector for production of the linear covalently closed DNA, such as a gene encoding AmpR (ampR) can be used. For the gene encoding AmpR (ampR), primer 11 (SEQ ID NO: 21) and primer 12 (SEQ ID NO: 22) are used as primers for real-time PCR.

In Comparative Example 1 described later, HEK293T cells were transfected with a circular plasmid DNA to produce an adeno-associated virus vector of the Parvoviridae family, with which in the supernatant-derived sample (culture supernatant), the number of vector genomes of the sequences (unnecessary sequences) other than the nucleic acid sequence encoding a protein was 14.4% relative to the number of the vector genomes of the nucleic acid sequence encoding a protein.

In Comparative Example 1 described later, moreover, HEK293T cells were transfected with a circular plasmid DNA to produce an adeno-associated virus vector of the Parvoviridae family, with which, in the cell-derived sample (cell solution), the number of vector genomes of the sequences (unnecessary sequences) other than the nucleic acid sequence encoding a protein was 1.2% relative to the number of the vector genomes of the nucleic acid sequence encoding a protein.

In Examples 2-8 and 2-9 described later, on the other hand, HEK293T cells were transfected with the obtained linear covalently closed DNA to produce an adeno-associated virus vector of the Parvoviridae family, with which, both in the supernatant-derived sample (culture supernatant) and in the cell-derived sample (cell solution), the vector genomes of sequences (unnecessary sequences) other than the nucleic acid sequence encoding a protein were not detected at all (the number being equal to or less than the detection limit).

### <Another step B>

The above-mentioned another step B is not particularly limited, and may be appropriately selected according to the intended purpose. Examples of another step B include an expression induction step of inducing expression of the protelomerase after the gene introduction step, a purification step of a linear covalently closed DNA, and the like.

The expression induction step of inducing expression of the protelomerase after the gene introduction step is not particularly limited, and may be appropriately selected according to the intended purpose. Examples of the expression induction step include a method of adding an expression inducer to a fluid culture medium of a gene-introduced first host, and the like.

In the case where an arabinose-inducible promoter is used as the nucleic acid sequence regulating expression of the protelomerase, arabinose can be used as the expression inducer.

The purification step of the linear covalently closed DNA is not particularly limited, and may be appropriately selected according to the intended purpose. Examples of the purification step include: a method of purifying using a commercially available kit; a method of purifying a cell solution by cation exchange chromatography, anion exchange chromatography, or size exclusion chromatography, or using a filter or ultrafiltration membrane; and the like.

### (Parvovirus vector)

The parvovirus vector is produced by the above-described parvovirus vector production method.

The parvovirus vector can be used as a cellular or gene therapy agent, vaccine, and a vector for a therapeutic agent.

The parvovirus vector may be used as it is. The parvovirus vector may be modified in advance by post-translational modification, such as phosphorylation, methylation, lipid modification, glycosylation, deamidation, and the like. Alternatively, after the production of the parvovirus vector, modification to impart a pharmacological change, such as glycosylation, low molecular weight compound addition, PEGylation, and the like, or modification to impart a function, such as of an enzyme, of an isotope, and the like, may be carried out for use. Moreover, various types of formulation processing may be carried out.

### (Parvovirus-vector-producing cell)

The parvovirus-vector-producing cell is a cell that produces a parvovirus vector.

The parvovirus vector is a parvovirus vector that is obtained using a linear covalently closed DNA and expresses a nucleic acid sequence encoding a protein. The number of vector genomes of sequences other than the nucleic acid sequence encoding the protein relative to the number of vector genomes of the nucleic acid sequence encoding the protein, as quantified by quantitative PCR, is 10% or less in a culture supernatant of the parvovirus-vector-producing cells, or 1% or less in a cell solution of the parvovirus-vector-producing cells.

The parvovirus-vector-producing cell is obtained by transfecting the second host with the linear covalently closed DNA obtained by the above-described gene introduction step.

The gene introduction step, the second host, the transfection, the number of vector genomes of sequences (unnecessary sequences) other than the nucleic acid sequence encoding a protein quantified by the quantitative PCR, and the measuring method thereof are as described above.

### [Examples]

Examples of the present invention will be explained hereinafter but the present invention shall not be limited by these examples in any way.

The details of the operation methods and the like of the recombinant DNA technology used in Examples below are disclosed in the following literature: Molecular Cloning 2nd Edition (Cold Spring Harbor Laboratory Press, 1989), Current Protocols in Molecular Biology (Greene Publishing Associates and Wiley-Interscience).

<Production Example 1-1: preparation of various genes used for preparation of vector>

A nucleic acid sequence (SEQ ID NO: 1) encoding protelomerase (TelN protelomerase) derived from Escherichia virus N15, which was used for construction of a vector, was prepared by PCR using a synthetic DNA as a template. The synthetic DNA is produced by a method where a plurality of oligonucleotides designed to have sequences overlapping one another are synthesized, and after annealing, the oligonucleotides are extended to a target strand length using DNA ligase or DNA polymerase, or the like. The synthetic DNA can be also obtained by using artificial gene synthesis services provided by various companies.

As a pair of nucleic acid sequences (telRL sequence) recognized by protelomerase, which were used for construction of a vector, nucleic acid fragments (SEQ ID NO: 2) were obtained by total synthesis.

An AraC gene (SEQ ID NO: 3) regulated by a promoter, which was used for construction of a vector, was prepared by PCR using a synthetic DNA as a template. The synthetic DNA is produced by a method where a plurality of oligonucleotides designed to have sequences overlapping one another are synthesized, and after annealing, the oligonucleotides are extended to a target strand length using DNA ligase or DNA polymerase, or the like. The synthetic DNA can be also obtained by using artificial gene synthesis services provided by various companies.

As an arabinose-inducible promoter, which was used for construction of a vector, a nucleic acid fragment (SEQ ID NO: 4) was obtained by total synthesis.

A nucleic acid sequence (SEQ ID NO: 5) encoding a fluorescent protein (Venus), which was used for construction of a vector, was prepared by PCR using a synthetic DNA as a template. The synthetic DNA is produced by a method where a plurality of oligonucleotides designed to have sequences overlapping one another are synthesized, and after annealing, the oligonucleotides are extended to a target strand length using DNA ligase or DNA polymerase, or the like. The synthetic DNA can be also obtained by using artificial gene synthesis services provided by various companies.

A nucleic acid sequence encoding TelN protelomerase was prepared by PCR using primer 1 (SEQ ID NO: 6 forward primer) and primer 2 (SEQ ID NO: 7 reverse primer); an AraC gene regulated by a promoter was prepared by PCR using primer 3 (SEQ ID NO: 8 forward primer) and primer 4 (SEQ ID NO: 9 reverse primer); and a nucleic acid sequence encoding a fluorescent protein (Venus) was prepared by PCR using primer 5 (SEQ ID NO: 10 forward primer) and primer 6 (SEQ ID NO: 11 reverse primer).

PrimeSTAR Max DNA Polymerase (produced by TAKARA BIO INC.) or the like was used to perform the PCR in the reaction conditions according to the method described in the attached manual.

### <Example 1-1: construction of vector for production of linear covalently closed DNA>

In Example 1-1, a double-stranded circular plasmid used for transformation of Escherichia coli was prepared by introducing a constructed vector into E.coli DH5α competent cells (9057, available from TAKARA BIO INC.), and culturing and amplifying the obtained transformant.

Preparation of plasmid from a plasmid-carrying strain was carried out using FastGene Plasmid Mini Kit (produced by NIPPON Genetics Co., Ltd.).

For the nucleic acid sequence (SEQ ID NO: 5) encoding a fluorescent protein (Venus), a nucleic acid fragment was prepared by PCR using primer 5 (SEQ ID NO: 10) and primer 6 (SEQ ID NO: 11), and a green fluorescent protein (GFP) of pAAV-GFP Control Plasmid (produced by Cell Biolabs Inc.) was replaced with the prepared nucleic acid fragment to construct pAAV-Venus.

Next, nucleic acid fragments (SEQ ID NO: 2) as a pair of nucleic acid sequences (telRL sequence) recognized by protelomerase were prepared by total synthesis, and the prepared nucleic acid fragments were inserted into the PciI site and the KasI site of the pAAV-Venus, respectively, to construct pAAV-Venus_telRL.

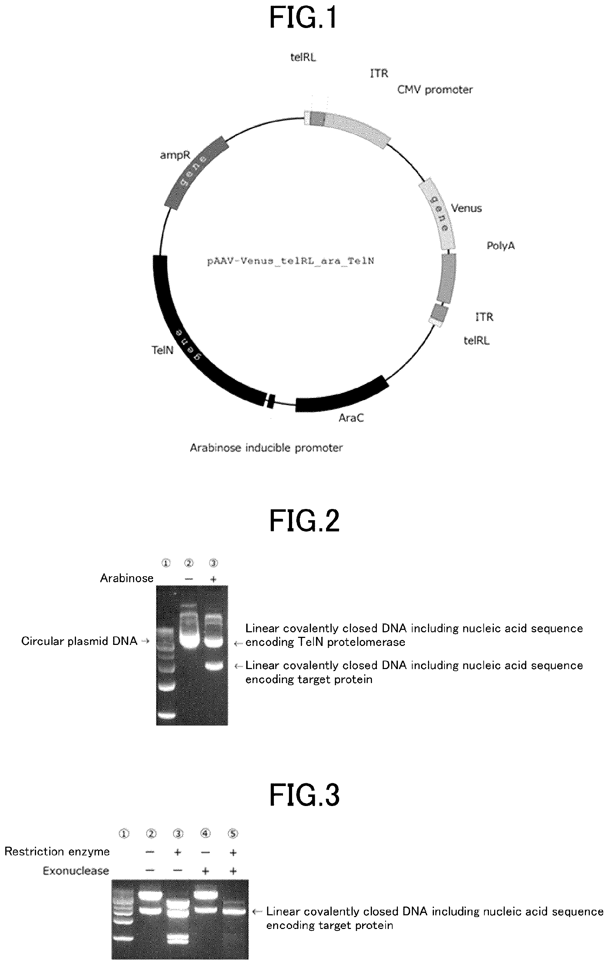

Next, a nucleic acid sequence (SEQ ID NO: 1) encoding TelN protelomerase was prepared as a nucleic acid fragment by PCR using primer 1 (SEQ ID NO: 6) and primer 2 (SEQ ID NO: 7), an AraC gene (SEQ ID NO: 3) regulated by a promoter was prepared as a nucleic acid fragment by PCR using primer 3 (SEQ ID NO: 8) and primer 4 (SEQ ID NO: 9), and a nucleic acid fragment (SEQ ID NO: 4) of an arabinose-inducible promoter was prepared by total synthesis, and the prepared nucleic acid fragments were all inserted into the PsiI site of the pAAV-Venus_telRL to construct pAAV-Venus_telRL_ara_TelN (Fig. 1).

The pAAV-Venus_telRL_ara_TelN vector is a vector for production of a linear covalently closed DNA, and is designed such that TelN protelomerase is expressed under regulation of the arabinose-inducible promoter. Moreover, the telRL sequences are used as the pair of nucleic acid sequences recognized by protelomerase, and the nucleic acid sequence encoding a fluorescent protein (Venus) serving as the nucleic acid sequence encoding a target protein is located between the telRL sequences.

The telRLs of Fig. 1 are the pair of nucleic acid sequences (telRL sequence) recognized by protelomerase. The ITR of Fig. 1 is an inverted terminal repeat. The CMV Promoter of Fig. 1 is a cytomegalovirus-derived promoter. The Venus of Fig. 1 is the nucleic acid sequence encoding a fluorescent protein (Venus). The PolyA of Fig. 1 is a sequence associated with polyadenylation of mRNA. The AraC of Fig. 1 is the AraC gene sequence regulated by a promoter. The Arabinose inducible Promoter of Fig. 1 is the nucleic acid sequence of the arabinose-inducible promoter. The TelN of Fig. 1 is the nucleic acid sequence encoding TelN protelomerase. The ampR of Fig. 1 is the nucleic acid sequence encoding an antibiotic resistant protein (AmpR).

### <Example 1-2: acquisition of transformed E. coli>

*E. coli* was transformed with pAAV-Venus_telRL_ara_TelN, the vector for production of a linear covalently closed DNA, which was constructed in Example 1-1, in the following manner.

A solution (25 µL) of competent cells of *E. coli* strain NEB10β (C3019H, produced by New England Biolabs, Inc.) and 100 pg of the pAAV-Venus_telRL_ara_TelN were mixed, and the mixture was left to stand on ice cubes for 30 minutes.

After standing for 30 minutes, a heat treatment was carried out at 42°C for 45 seconds, followed by leaving to stand on ice cubes for 2 minutes.

After the standing for 2 minutes, 225 µL of a SOC medium was added, and the *E. coli* was applied to a LB agar medium (1% tryptone, 0.5% dried yeast extract, 1% sodium chloride, 0.005% carbenicillin disodium salt (produced by NACALAI TESQUE, INC.)). Then, the cell clone that grew by static culture at 37°C for one day was selected to acquire *E. coli* into which pAAV-Venus_telRL_ara_TelN, the vector for production of a linear covalently closed DNA, was introduced.

### <Example 1-3: incubation of transformed Escherichia coli>

The transformed *E. coli* obtained in Example 1-2 was inoculated to 2 mL of a Plusgrow II medium (4% Plusgrow II (produced by NACALAI TESQUE, INC.), 0.005% carbenicillin disodium salt), followed by shake culture at 37°C for 6 hours, to obtain a pre-culture fluid medium.

In 50 mL of a Plusgrow II medium (4% Plusgrow II, 0.005% carbenicillin disodium salt), 100 µL of the pre-culture fluid medium was subcultured, followed by shake culture at 37°C for 16 hours.

After the shake culture for 16 hours, 50 mL of a Plusgrow II medium (4% Plusgrow II, 0.005% carbenicillin disodium salt) and 1 mL of an arabinose solution (10% arabinose) were added, and the resulting mixture was shake cultured at 37°C for 1 hour. After the shake culture for 1 hour, centrifugal separation was carried out to collect the bacteria.

### <<Evaluation of linear covalently closed DNA by electrophoresis>>

Evaluation of the linear covalently closed DNA prepared from the *E. coli* bacteria obtained in Example 1-3 was carried out by electrophoresis in the following manner.

The preparation of DNA from the *E. coli* bacteria was carried out using FastGene Plasmid Mini Kit (produced by NIPPON Genetics Co., Ltd.). The obtained DNA solution was applied into wells of 1% agar gel produced with a TAE buffer, and electrophoresis was carried out at 100 V for 40 minutes. A nucleic acid dye was used for dying, and UV light was used for detection. The result is presented in LANE 3 of Fig. 2.

The evaluation result of DNA, which was obtained from the *E. coli* bacteria in the same manner except that 1 mL of the arabinose solution was not added, is presented in LANE 2 of Fig. 2.

According to the results of Fig. 2, two bands derived from the linear covalently closed DNAs (the band derived from the linear covalently closed DNA including the nucleic acid sequence encoding a target protein and the band derived from the linear covalently closed DNA including the nucleic acid sequence encoding TelN protelomerase) were confirmed from the *Escherichia coli* bacteria derived from the fluid culture medium to which arabinose was added (LANE 3), whereas a band derived from the linear covalently closed DNA was not confirmed and only the band derived from the circular plasmid DNA was confirmed from the *Escherichia coli* bacteria derived from the fluid culture medium to which arabinose was not added (LANE 2). LANE 1 of Fig. 2 depicts a DNA marker (1 kb DNA Ladder, produced by TAKARA BIO INC., 3412A).

The results indicate that the TelN protelomerase cleaved and rebound the telRL sequences recognized by the TelN protelomerase to thereby produce a linear covalently closed DNA including the nucleic acid sequence encoding a target protein, which was originally located between the telRL sequences.

### <Example 1-4: purification of linear covalently closed DNA>

The linear covalently closed DNA obtained in Example 1-3 was processed with restriction enzymes EcoRV and BclI, and the linear covalently closed DNA was purified using NucleoSpin Gel and PCR Clean-up Kit (produced by MACHEREY-NAGEL GmbH & Co. KG.). As a result, the linear covalently closed DNA having a nucleic acid sequence encoding TelN protelomerase and the vector for production of the linear covalently closed DNA were cleaved at two sites by the restriction enzymes.

Next, the linear covalently closed DNA was processed with Exonuclease (produced by New England Biolabs, Inc.), and the linear covalently closed DNA was purified using NucleoSpin Gel and PCR Clean-up Kit (produced by MACHEREY-NAGEL GmbH & Co. KG.). As a result, the nucleotides of the ends of a single-stranded or double-stranded DNA were decomposed.

### <<Evaluation of linear covalently closed DNA by electrophoresis>>

Evaluation of the linear covalently closed DNA obtained in Example 1-4 was carried out by electrophoresis in the following manner.

The obtained DNA solution was applied into wells of 1% agar gel produced with TAE buffer, and electrophoresis was carried out at 100 V for 40 minutes. A nucleic acid dye was used for dying, and UV light was used for detection. The results are presented in Fig. 3 (LANEs 3, 4, and 5).

LANE 1 of Fig. 3 depicts the DNA marker (1 kb DNA Ladder, produced by TAKARA BIO INC., 3412A); LANE 2 depicts the sample not processed with the restriction enzymes, nor Exonuclease; LANE 3 depicts the sample processed with the restriction enzymes but not with Exonuclease; LANE 4 depicts the sample not processed with the restriction enzymes but with Exonuclease; and LANE 5 depicts the sample processed with both the restriction enzymes and Exonuclease.

According to the results of Fig. 3, decomposition due to Exonuclease was not confirmed with LANE 4, and only the band derived from the linear covalently closed DNA having the nucleic acid encoding the target protein located between telRL sequences was observed with LANE 5. Therefore, the results suggest that the restriction enzyme cleaved the linear covalently closed DNA having the nucleic acid sequence encoding TelN protelomerase, and Exonuclease decomposed the nucleotides present at the ends of single-stranded or double-stranded DNA. Namely, the results indicate that the ends of the linear covalently closed DNA obtained in Example 1-3 were surely closed.

### <Example 1-5: construction of vector for production of linear covalently closed DNA>

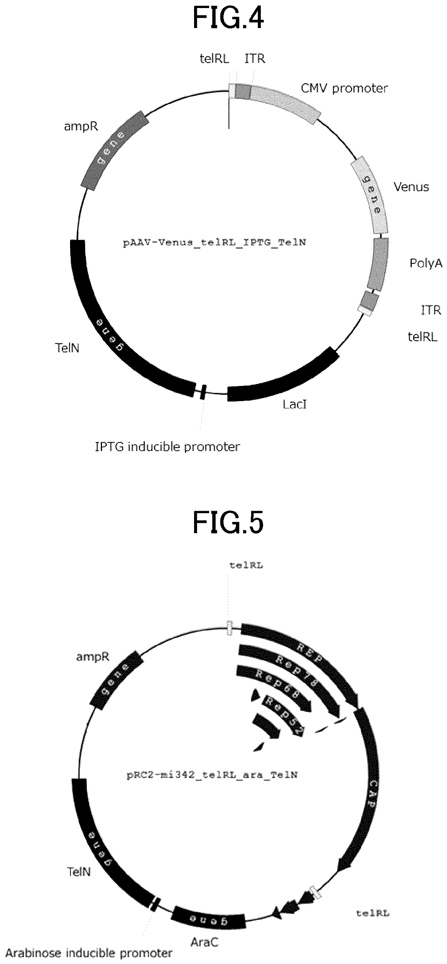

A nucleic acid sequence (SEQ ID NO: 1) encoding TelN protelomerase was prepared as a nucleic acid fragment by PCR using primer 1 (SEQ ID NO: 6) and primer 2 (SEQ ID NO: 7), and a LacI gene (SEQ ID NO: 23) regulated by a promoter and a nucleic acid fragment (SEQ ID NO: 24) of an IPTG-inducible promoter were prepared by total synthesis. The prepared nucleic acid fragments were inserted into the PsiI site of the pAAV-Venus_telRL constructed in Example 1-1 to construct pAAV-Venus_telRL_IPTG_TelN (Fig. 4).

The pAAV-Venus_telRL_IPTG_TelN vector is a vector for production of a linear covalently closed DNA, and is designed such that TelN protelomerase is expressed under regulation of the IPTG-inducible promoter. Moreover, the telRL sequences are used as the pair of nucleic acid sequences recognized by protelomerase, and the nucleic acid sequence encoding a fluorescent protein (Venus) serving as the nucleic acid sequence encoding a target protein is located between the telRL sequences.

The telRL of Fig. 4 is the pair of nucleic acid sequences (telRL sequences) recognized by protelomerase. The ITR of Fig. 4 is an inverted terminal repeat. The CMV Promoter of Fig. 4 is the cytomegalovirus-derived promoter. The Venus of Fig. 4 is the nucleic acid sequence encoding a fluorescent protein (Venus). The PolyA of Fig. 4 is a sequence associated with polyadenylation of mRNA. The LacI of Fig. 4 is the LacI gene sequence regulated by the promoter. The IPTG inducible Promoter of Fig. 4 is the nucleic acid sequence of the IPTG-inducible promoter. The TelN of Fig. 4 is the nucleic acid sequence encoding TelN protelomerase. The ampR of Fig. 4 is the nucleic acid sequence encoding the antibiotic resistant protein (AmpR).

### <Example 1-6: acquisition of transformed E. coli>

*E. coli* was transformed using pAAV-Venus_telRL_IPTG_TelN, the vector for production of a linear covalently closed DNA, which was constructed in Example 1-5, in the following manner.

Fifty microliters of a solution of competent cells of *E*. *coli* strain JM109 (9052, produced by TAKARA BIO INC.) or a solution of competent cells of the DH5α strain (9057, produced by TAKARA BIO INC.) and 100 pg of pAAV-Venus_telRL_IPTG_TelN were mixed, and the resulting mixture was left to stand on ice cubes for 30 minutes.

After the standing for 30 minutes, a heat treatment was carried out at 42°C for 45 seconds, followed by leaving to stand on ice cubes for 2 minutes.

After the standing for 2 minutes, 450 µL of a SOC medium was added, and the *E. coli* was applied to a LB agar medium (1% tryptone , 0.5% dried yeast extract, 1% sodium chloride, 0.005% carbenicillin disodium salt (produced by NACALAI TESQUE, INC.)). Then, the cell clone that grew by static culture at 37°C for one day was selected to acquire *E. coli* into which pAAV-Venus_telRL_IPTG_TelN, the vector for production of a linear covalently closed DNA, was introduced.

In Examples 2-1, 2-2, 2-3, and 2-4 below, a plasmid used for transformation of *E. coli* was prepared by introducing the constructed vector into competent cells of *E. coli* DH5α (9057, produced by TAKARA BIO INC.), and culturing and amplifying the obtained transformant. The preparation of the plasmid from the plasmid-carrying strain was carried out using FastGene Plasmid Mini Kit (produced by NIPPON Genetics Co., Ltd.).

### <Production Example 2-1: preparation of genes used for preparation of vector>

A nucleic acid sequence (SEQ ID NO: 1) encoding protelomerase (TelN protelomerase) derived from Escherichia virus N15, which was used for construction of a vector, was prepared by PCR using a synthetic DNA as a template. The synthetic DNA is produced by a method where a plurality of oligonucleotides designed to have sequences overlapping one another are synthesized, and after annealing, the oligonucleotides are extended to a target strand length using DNA ligase or DNA polymerase, or the like. The synthetic DNA can be also obtained by using artificial gene synthesis services provided by various companies.

As a pair of nucleic acid sequences (telRL sequence) recognized by protelomerase, which was used for construction of a vector, nucleic acid fragments (SEQ ID NO: 2) were obtained by total synthesis.

An AraC gene (SEQ ID NO: 3) regulated by a promoter, which was used for construction of a vector, was prepared by PCR using a synthetic DNA as a template. The synthetic DNA is produced by a method where a plurality of oligonucleotides designed to have sequences overlapping one another are synthesized, and after annealing, the oligonucleotides are extended to a target strand length using DNA ligase or DNA polymerase, or the like. The synthetic DNA can be also obtained by using artificial gene synthesis services provided by various companies.

As an arabinose-inducible promoter, which was used for construction of a vector, a nucleic acid fragment (SEQ ID NO: 4) was obtained by total synthesis.

A nucleic acid sequence (SEQ ID NO: 5) encoding a fluorescent protein (Venus), which was used for construction of a vector, was prepared by PCR using a synthetic DNA as a template. The synthetic DNA is produced by a method where a plurality of oligonucleotides designed to have sequences overlapping one another are synthesized, and after annealing, the oligonucleotides are extended to a target strand length using DNA ligase or DNA polymerase, or the like. The synthetic DNA can be also obtained by using artificial gene synthesis services provided by various companies.

A nucleic acid sequence encoding TelN protelomerase was prepared by PCR using primer 1 (SEQ ID NO: 6 forward primer) and primer 2 (SEQ ID NO: 7 reverse primer); an AraC gene regulated by a promoter was prepared by PCR using primer 3 (SEQ ID NO: 8 forward primer) and primer 4 (SEQ ID NO: 9 reverse primer); and a nucleic acid sequence encoding a fluorescent protein (Venus) was prepared by PCR using primer 5 (SEQ ID NO: 10 forward primer) and primer 6 (SEQ ID NO: 11 reverse primer).

PrimeSTAR Max DNA Polymerase (produced by TAKARA BIO INC.) or the like was used to perform the PCR in the reaction conditions according to the method described in the attached manual.

### <Example 2-1: construction of vector having target gene>

For the nucleic acid sequence (SEQ ID NO: 5) encoding a fluorescent protein (Venus), a nucleic acid fragment was prepared by PCR using primer 5 (SEQ ID NO: 10) and primer 6 (SEQ ID NO: 11), and a green fluorescent protein (GFP) of pAAV-GFP Control Plasmid (produced by Cell Biolabs Inc.) was replaced with the prepared nucleic acid fragment to construct pAAV-Venus.

Next, nucleic acid fragments (SEQ ID NO: 2) as a pair of nucleic acid sequences (telRL sequence) by protelomerase were prepared by total synthesis, the prepared nucleic acid fragments were inserted into the PciI site and the KasI site of the pAAV-Venus, respectively, to construct pAAV-Venus_telRL.

Next, a nucleic acid sequence (SEQ ID NO: 1) encoding TelN protelomerase was prepared as a nucleic acid fragment by PCR using primer 1 (SEQ ID NO: 6) and primer 2 (SEQ ID NO: 7), an AraC gene (SEQ ID NO: 3) regulated by a promoter was prepared as a nucleic acid fragment by PCR using primer 3 (SEQ ID NO: 8) and primer 4 (SEQ ID NO: 9), and a nucleic acid fragment (SEQ ID NO: 4) of an arabinose-inducible promoter was prepared by total synthesis, and the prepared nucleic acid fragments were all inserted into the PsiI site of the pAAV-Venus_telRL to construct pAAV-Venus_telRL_ara_TelN (Fig. 1).

The pAAV-Venus_telRL_ara_TelN vector is a vector for production of a linear covalently closed DNA, and is designed such that TelN protelomerase is expressed under regulation of the arabinose-inducible promoter. Moreover, the telRL sequences are used as the pair of nucleic acid sequences recognized by protelomerase, and the nucleic acid sequence encoding a fluorescent protein (Venus) serving as the nucleic acid sequence encoding a target protein is located between the telRL sequences.

The telRL of Fig. 1 is the pair of nucleic acid sequences (telRL sequence) recognized by protelomerase. The ITR of Fig. 1 is an inverted terminal repeat. The CMV Promoter of Fig. 1 is the cytomegalovirus-derived promoter. The Venus of Fig. 1 is the nucleic acid sequence encoding a fluorescent protein (Venus). The PolyA of Fig. 1 is a sequence associated with polyadenylation of mRNA. The AraC of Fig. 1 is the AraC gene sequence regulated by a promoter. The Arabinose inducible Promoter of Fig. 1 is the nucleic acid sequence of the arabinose-inducible promoter. The TelN of Fig. 1 is the nucleic acid sequence encoding TelN protelomerase. The ampR of Fig. 1 is the nucleic acid sequence encoding the antibiotic resistant protein (AmpR).

### <Example 2-2: construction of vector having packaging gene>

Nucleic acid fragments (SEQ ID NO: 2) as a pair of nucleic acid sequences (telRL sequence) recognized by protelomerase were prepared by total synthesis, the prepared nucleic acid fragments were inserted into the EcoRV site and the SnaBI site of pRC2-mi342 (produced by TAKARA BIO INC.), respectively, to construct pRC2-mi342_telRL.

Next, a nucleic acid sequence (SEQ ID NO: 1) encoding TelN protelomerase was prepared as a nucleic acid fragment by PCR using primer 1 (SEQ ID NO: 6) and primer 2 (SEQ ID NO: 7), an AraC gene (SEQ ID NO: 3) regulated by a promoter was prepared as a nucleic acid fragment by PCR using primer 3 (SEQ ID NO: 8) and primer 4 (SEQ ID NO: 9), and a nucleic acid fragment (SEQ ID NO: 4) of an arabinose-inducible promoter was prepared by total synthesis, and the prepared nucleic acid fragments were all inserted into the SmaI site of pRC2-mi342_telRL to construct pRC2-mi342_telRL_ara_TelN (Fig. 5).

The pRC2-mi342_telRL_ara_TelN vector is a vector for production of a linear covalently closed DNA, and is designed such that TelN protelomerase is expressed under regulation of the arabinose-inducible promoter. Moreover, the telRL sequences were used as the pair of nucleic acid sequences recognized by protelomerase, and the nucleic acid sequence (Rep: SEQ ID NO: 12, Cap: SEQ ID NO: 13) encoding a packaging protein is located between the telRL sequences.

The Rep of Fig. 5 is the nucleic acid sequence encoding the packaging protein Rep of the adeno-associated virus. The Cap of Fig. 5 is the nucleic acid sequence encoding the packaging protein Cap of the adeno-associated virus.

### <Example 2-3: construction of vector having helper gene>

Nucleic acid fragments (SEQ ID NO: 2) as a pair of nucleic acid sequences (telRL sequence) recognized by protelomerase were prepared by total synthesis, the prepared nucleic acid fragments were inserted into the BamHI site and the SalI site of pHelper (produced by TAKARA BIO INC.), respectively, to construct pHelper_telRL.

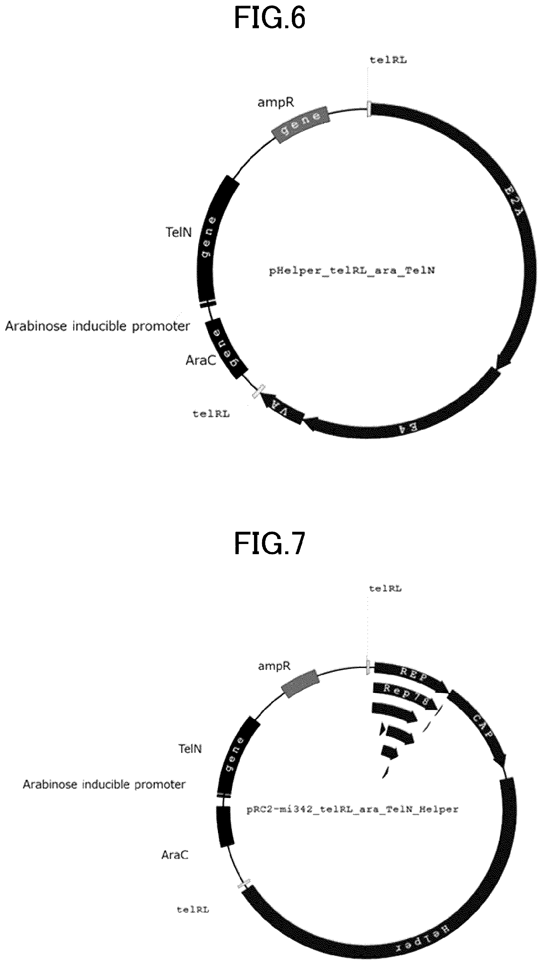

Next, a nucleic acid sequence (SEQ ID NO: 1) encoding TelN protelomerase was prepared as a nucleic acid fragment by PCR using primer 1 (SEQ ID NO: 6) and primer 2 (SEQ ID NO: 7), an AraC gene (SEQ ID NO: 3) regulated by a promoter was prepared as a nucleic acid fragment by PCR using primer 3 (SEQ ID NO: 8) and primer 4 (SEQ ID NO: 9), and a nucleic acid fragment (SEQ ID NO: 4) of an arabinose-inducible promoter was prepared by total synthesis, and the prepared nucleic acid fragments were all inserted into the NdeI site of the pHelper_telRL to construct pHelper_telRL_ara_TelN (Fig. 6).

The pHelper_telRL_ara_TelN vector is a vector for production of a linear covalently closed DNA, and is designed such that TelN protelomerase is expressed under regulation of arabinose-inducible promoter. Moreover, the telRL sequences are used as the pair of nucleic acid sequences recognized by protelomerase, and the nucleic acid sequence (E2A: SEQ ID NO: 14, E4: SEQ ID NO: 15, VA: SEQ ID NO: 16) encoding a helper protein is located between the telRL sequences.

The E2A of Fig. 6 is the nucleic acid sequence encoding the helper protein E2A of adenovirus. The E4 of Fig. 6 is the nucleic acid sequence encoding the helper protein E4 of adenovirus. The VA of Fig. 6 is the nucleic acid sequence encoding the helper protein VA of adenovirus.

### <Example 2-4: construction of vector having packaging gene and helper gene>

A nucleic acid sequence encoding a helper protein was prepared as a nucleic acid fragment by PCR using pHelper (produced by TAKARA BIO INC.) as a template and using primer 7 (SEQ ID NO: 17 forward primer) and primer 8 (SEQ ID NO: 18 reverse primer). The prepared nucleic acid sequence was inserted into the NdeI site of the pRC2-mi342_telRL_ara_TelN obtained in Example 2-2 to construct pRC2-mi342_telRL_ara_TelN_Helper (Fig. 7) .

The pRC2-mi342_telRL_ara_TelN_Helper vector is a vector for production of a linear covalently closed DNA, and is designed such that TelN protelomerase is expressed under regulation of the arabinose-inducible promoter. Moreover, the telRL sequences are used as the pair of nucleic acid sequences recognized by protelomerase, and the nucleic acid sequence encoding a packaging protein and helper protein is located between the telRL sequences.

The Helper of Fig. 7 is the nucleic acid sequence encoding the helper proteins E2A, E4, and VA of adenovirus.

### <Example 2-5: acquisition of transformed E. coli>

*E. coli* was transformed with pAAV-Venus_telRL_ara_TelN that was the vector for production of linear covalently closed DNA constructed in Example 2-1, pRC2-mi342_telRL_ara_TelN that was the vector for production of linear covalently closed DNA constructed in Example 2-2, pHelper_telRL_ara_TelN that was the vector for production of linear covalently closed DNA constructed in Example 2-3, or pRC2-mi342_telRL_ara_TelN_Helper that was the vector for production of linear covalently closed DNA constructed in Example 2-4, in the following manner.

With 25 µL of a solution of competent cells of *E. coli* strain NEB10β (C3019H, produced by New England Biolabs, Inc.), a linear covalently closed DNA production vector solution including 100 pg of the pAAV-Venus_telRL_ara_TelN, 100 pg of the pRC2-mi342_telRL_ara_TelN, 100 pg of the pHelper_telRL_ara_TelN, or 100 pg of the pRC2-mi342_telRL_ara_TelN_Helper was mixed, and the resulting mixture was left to stand on ice cubes for 30 minutes.

After the standing for 30 minutes, a heat treatment was carried out at 42°C for 45 seconds, followed by leaving to stand on ice cubes for 2 minutes.

After the standing for 2 minutes, 225 µL of a SOC medium was added, and the *E. coli* was applied to a LB agar medium (1% tryptone, 0.5% dried yeast extract, 1% sodium chloride, 0.005% carbenicillin disodium salt (produced by NACALAI TESQUE, INC.)). Then, the cell clone that grew by static culture at 37°C for one day was selected to acquire *E. coli* into which the vector for producing a linear covalently closed DNA was introduced.

### <Example 2-6: incubation of transformed Escherichia coli>

The transformed *E. coli* obtained in Example 2-5 was seeded to 2 mL of a Plusgrow II medium (4% Plusgrow II (produced by NACALAI TESQUE, INC.), 0.005% carbenicillin disodium salt), followed by shake culture at 37°C for 6 hours, to obtain a pre-culture fluid medium.

In 50 mL of a Plusgrow II medium (4% Plusgrow II, 0.005% carbenicillin disodium salt), 100 µL of the pre-culture fluid medium was subcultured, followed by shake culture at 37°C for 16 hours.

After the shake culture for 16 hours, 50 mL of a Plusgrow II medium (4% Plusgrow II, 0.005% carbenicillin disodium salt) and 1 mL of an arabinose solution (10% arabinose) were added, and the resulting mixture was shake cultured at 37°C for 1 hour. After the shake culture for 1 hour, centrifugal separation was carried out to collect the bacteria.

### <<Evaluation of linear covalently closed DNA by electrophoresis>>

Evaluation of the linear covalently closed DNA prepared from the *E. coli* bacteria obtained in Example 2-6 was carried out by electrophoresis in the following manner.

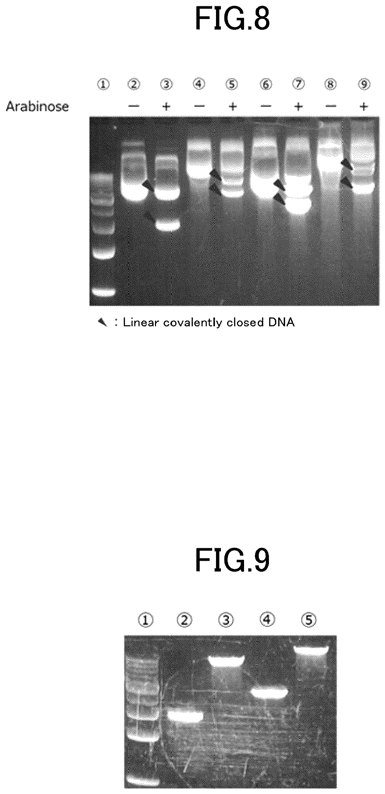

The preparation of DNA from the *E. coli* bacteria was carried out using FastGene Plasmid Mini Kit (produced by NIPPON Genetics Co., Ltd.). The obtained DNA solution was applied into wells of 1% agar gel produced with TAE buffer, and electrophoresis was carried out at 100 V for 40 minutes. A nucleic acid dye was used for dying, and UV light was used for detection. The result is presented in LANEs 3, 5, 7, and 9 of Fig. 8.

The evaluation results of DNA, which was obtained from the *E. coli* bacteria incubated in the same manner except that 1 mL of the arabinose solution was not added, are presented in LANEs 2, 4, 6, and 8 of Fig. 8.

LANE 1 of Fig. 8 depicts the DNA marker (1 kb DNA Ladder, produced by TAKARA BIO INC., 3412A), LANE 3 depicts the pAAV-Venus _telRL_ara _TelN-derived sample, LANE 5 depicts the pHelper_telRL_ara_TelN-derived sample, LANE 7 depicts the pRC2-mi342_telRL_ara_TelN-derived sample, LANE 9 depicts the pRC2-mi342_telRL_ara_TelN_Helper-sample, LANE 2 depicts the pAAV-Venus _telRL_ara _TelN-derived sample to which the arabinose solution was not added, LANE 4 depicts the pHelper_telRL_ara_TelN-derived sample to which the arabinose solution was not added, LANE 6 depicts the pRC2-mi342_telRL_ara _TelN-derived sample to which the arabinose solution was not added, and LANE 8 depicts the pRC2-mi342_telRL_ara_TelN_Helper-derived sample to which the arabinose solution was not added.

According to the results of Fig. 8, two bands derived from the linear covalently closed DNAs (the band derived from the linear covalently closed DNA including the nucleic acid sequence encoding a target protein, the band derived from the linear covalently closed DNA including the nucleic acid sequence encoding a packaging protein, the band derived from the linear covalently closed DNA including the nucleic acid sequence encoding a helper protein, or the band derived from the linear covalently closed DNA including the nucleic acid sequence encoding a helper protein, and the band derived from the linear covalently closed DNA including the nucleic acid sequence encoding TelN protelomerase, as indicated with the arrows in Fig. 8) were confirmed from the *E. coli* bacteria derived from the fluid culture medium to which arabinose was added (LANEs 3, 5, 7, and 9), whereas the band derived from the linear covalently closed DNA was not confirmed and only the band derived from the circular plasmid DNA was confirmed from the Escherichia coli bacteria derived from the fluid culture medium to which the arabinose was not added (LANE 2, 4, 6, 8).

The results indicate that, as the vector including the nucleic acid sequence encoding protelomerase, the pair of nucleic acid sequences recognized by the protelomerase, and the nucleic acid sequence located between the pair of the nucleic acid sequences and encoding a protein was introduced into the *E*. *coli* serving as the first host, the TelN protelomerase cleaved and rejoined the vector at the sites of the telRL sequences recognized by the TelN protelomerase to thereby produce the linear covalently closed DNA including the nucleic acid sequence encoding a target protein that was originally located between the telRL sequences, the nucleic acid sequence encoding a packaging protein that was originally located between the telRL sequence, the nucleic acid sequence encoding a helper protein that was originally located between the telRL sequences, or the nucleic acid sequence encoding a packaging protein and a helper protein that was originally located between the telRL sequences.

### <Example 2-7: purification of linear covalently closed DNA>

The linear covalently closed DNA obtained in Example 2-6 was processed with a restriction enzyme BstZ17I (produced by New England Biolabs, Inc.), and the linear covalently closed DNA was purified using NucleoSpin Gel and PCR Clean-up Kit (produced by MACHEREY-NAGEL GmbH & Co. KG.). As a result, the linear covalently closed DNA having the nucleic acid sequence encoding TelN protelomerase and the vector for production of a linear covalently closed DNA were cleaved at the BstZ17I recognition sites thereof.

Next, the linear covalently closed DNA was processed with Exonuclease (produced by New England Biolabs, Inc.), and the linear covalently closed DNA was purified using NucleoSpin Gel and PCR Clean-up Kit (produced by MACHEREY-NAGEL GmbH & Co. KG.). As a result, the nucleotides of the ends of the single-stranded or double-stranded DNA were decomposed.

### <<Evaluation of linear covalently closed DNA by electrophoresis>>

Evaluation of the linear covalently closed DNA obtained in Example 2-7 was carried out by electrophoresis in the following manner.

The obtained DNA solution was applied into wells of 1% agar gel produced with TAE buffer, and electrophoresis was carried out at 100 V for 40 minutes. A nucleic acid dye was used for dying, and UV light was used for detection. The results are presented in Fig. 9 (LANEs 2, 3, 4, and 5).

The linear covalently closed DNA having the nucleic acid sequence encoding a target protein located between the telRL sequences is Venus_LCC_DNA; the linear covalently closed DNA having the nucleic acid sequence encoding a packaging protein located between the telRL sequences is RC2_LCC_DNA; the linear covalently closed DNA having the nucleic acid sequence encoding a helper protein located between the telRL sequences is Helper_LCC_DNA; and a linear covalently closed DNA having the nucleic acid sequence encoding a packaging protein and a helper protein located between the telRL sequences is RC2_Helper_LCC_DNA.

LANE 1 of Fig. 9 depicts the DNA marker (1 kb DNA Ladder, produced by TAKARA BIO INC., 3412A); LANE 2 depicts Venus_LCC_DNA; LANE 3 depicts Helper_LCC_DNA; LANE 4 depicts RC2_LCC_DNA; and LANE 5 depicts RC2_Helper_LCC_DNA.

According to the results of Fig. 9, the bands derived from the linear covalently closed DNAs each including the nucleic acid sequence encoding a protein located between the telRL sequence were confirmed, respectively, whereas a band derived from the linear covalently closed DNA including the nucleic acid sequence encoding the TelN protelomerase and a band derived from the vector for production of a linear covalently closed DNA were not confirmed.

The results indicated that the restriction enzyme BstZ17I cleaved the nucleic acid sequence encoding TelN protelomerase, and the Exonuclease decomposed the nucleotides present at the ends of single-stranded or double-stranded DNA. Namely, the results indicated that the ends of the linear covalently closed DNA obtained in Example 2-6 were surely closed.

### <Example 2-8: production of adeno-associated virus vector>

An adeno-associated virus vector was produced using the linear covalently closed DNAs obtained in Example 2-7 in the following manner.

HEK293T cells (obtained from ATCC) were seeded on a 10 cm culture dish to be 6.3×10⁵ cells, followed by incubating in the atmosphere of 5% CO₂ at 37°C for 3 days.

Three days later, 6 µg of Venus_LCC_DNA, 6 µg of RC2_LCC_DNA, and 12 µg of Helper_LCC_DNA were mixed to prepare Liquid A. A solution including 72 µg of PEI (produced by Polysciences) was provided as Liquid B. Liquid A and Liquid B were mixed, and the resulting mixture was left to stand at room temperature for 15 minutes.

To the HEK293T cells on a fresh medium, the mixture of Liquid A and Liquid B was added. The resulting mixture was incubated in the atmosphere of 5% CO₂ at 37°C for 4 days.

Four days later, the fluid culture medium of the culture dish was collected. To the culture dish, 3 mL of a PBS solution was added, and then the PBS solution was collected. To the culture dish, 1 mL of an Accutase solution (produced by Innovative Cell Technologies, Inc.) was added to separate the cells, and the cell suspension liquid was collected. To the cell suspension liquid, 6 mL of a PBS solution was added, and then collected. The collected fluid was subjected to centrifugal separation to collect a supernatant and cells.

### <<Measurement evaluation of number of vector genomes of adeno-associated virus vector by quantitative PCR>>

Measurement evaluation of the number of vector genomes of the adeno-associated virus vector obtained in Example 2-8 was carried out by real-time PCR, which was one of quantitative PCR methods, in the following manner.

To the supernatant, 8 mL of a 2.5 M NaCl solution including 40% PEG8000 (produced by Sigma-Aldrich) was added, and the resulting mixture was left to stand on ice cubes for 2 hours. After performing centrifugal separation, the supernatant was removed, and 1 mL of a PBS solution including 0.1% Triton X100 was added to the pellet. The resulting mixture was stirred at 4°C for 20 minutes. To the mixture, 1 µL of a KANEKA Endonuclease (KEN02500, produced by KANEKA CORPORATION, >250 U/uL) solution and 7.5 µL of a 1 M MgCl₂ solution were added, and the resulting mixture was left to stand at 37°C for 30 minutes. After the standing, 15 µL of a 0.5 M EDTA solution was added. Centrifugal separation was carried out, then the supernatant was collected to prepare a supernatant-derived sample (culture supernatant).

To the cells, 1 mL of a PBS solution including 0.1% Triton X100 was added, and the resulting mixture was stirred at 4°C for 20 minutes. To the mixture, 1 µL of a solution of KANEKA Endonuclease (KEN02500, produced by KANEKA CORPORATION, >250 U/µL) and 7.5 µL of a 1 M MgCl₂ solution were added, and the resulting mixture was left to stand at 37°C for 30 minutes. After the standing, 15 µL of a 0.5 M EDTA solution was added. Centrifugal separation was carried out, then the supernatant was collected to prepare a cell-derived sample (cell solution).

Real-time PCR (Quantstudio, SYBR Green) was carried out using the obtained supernatant-derived sample and cell-derived sample to measure the number of vector genomes of the nucleic acid sequence (target gene) encoding a target protein. As primers, primer 9 (SEQ ID NO: 19 forward primer) and primer 10 (SEQ ID NO: 20 reverse primer) were used. The results (vg/dish) of the number of vector genomes of the adeno-associated virus vector per culture dish are presented in Table 1.

**[Table 1]**

| | Vector | Supernatant-derived sample (vg/dish) | Cell-derived sample (vg/dish) |
|---|---|---|---|
| Example 8 | Venus_LCC_DNA, RC2_LCC_DNA, Helper_LCC_DNA | 4.09±1.28-10^11 | 6.89±0.37×10^11 |
| Example 9 | Venus_LCC_DNA, RC2_Helper_LCC_DNA | 1.82±0.32×10^11 | 3.38±0.11×10^11 |
| Comparative Example 1 | pAAV-Venus, pRC2-mi342, pHelper | 1.40±0.48×10^11 | 2.75±0.68×10^11 |

In order to evaluate erroneous packaging of the adeno-associated virus vector, moreover, the number of vector genomes of the gene (ampR) encoding an antibiotic resistant protein (AmpR), which was a sequence (unnecessary sequence) other than the nucleic acid sequences encoding proteins, was measured in the same manner as described above. As primers , primer 11 (SEQ ID NO: 21 forward primer) and primer 12 (SEQ ID NO: 22 reverse primer) were used. The results are presented in Table 2.

**[Table 2]**

| | Vector | Supernatant-derived sample (vg/dish) | Cell-derived sample (vg/dish) |
|---|---|---|---|
| Example 8 | Venus_LCC_DNA, RC2_LCC_DNA, Helper_LCC_DNA | ND | ND |
| Example 9 | Venus_LCC_DNA, RC2_Helper_LCC_DNA | ND | ND |
| Comparative Example 1 | pAAV-Venus, pRC2-mi342, pHelper | 2.02±0.50×10^10 | 3.42±1.28×10^9 |

| | | | |
|---|---|---|---|
| ND: Not detected | | | |

### <Example 2-9: production of adeno-associated virus vector>

An adeno-associated virus vector was produced using the linear covalently closed DNAs obtained in Example 2-7 in the following manner.

HEK293T cells (obtained from ATCC) were seeded on a 10 cm culture dish to be 6.3×10⁵ cells, followed by incubating in the atmosphere of 5% CO₂ at 37°C for 3 days.

Three day later, 8 µg of Venus_LCC_DNA and 16 µg of RC2_Helper_LCC_DNA were mixed to prepare Liquid A. A solution including 72 µg of PEI (produced by Polysciences) was provided as Liquid B. Liquid A and Liquid B were mixed, and the resulting mixture was left to stand at room temperature for 15 minutes.

To the HEK293T cells on a fresh medium, the mixture of Liquid A and Liquid B was added. The resulting mixture was incubated in the atmosphere of 5% CO₂ at 37°C for 4 days.

Four days later, the fluid culture medium of the culture dish was collected. To the culture dish, 3 mL of a PBS solution was added, and then the PBS solution was collected. To the culture dish, 1 mL of an Accutase solution (produced by Innovative Cell Technologies, Inc.) was added to separate the cells, and the cell suspension liquid was collected. To the cell suspension liquid, 6 mL of a PBS solution was added, and then collected. The collected fluid was subjected to centrifugal separation to collect cells and a supernatant.

Measurement evaluation of the number of vector genomes of the obtained adeno-associated virus vector was carried out by real-time PCR in the same manner as in Example 2-8. The results are presented in Tables 1 and 2.

### <Comparative Example 2-1: production of adeno-associated virus vector>

An adeno-associated virus vector was produced using circular plasmid DNA in the following manner.

HEK293T cells (obtained from ATCC) were seeded on a 10 cm culture dish to be 6.3×10⁵ cells, followed by incubating in the atmosphere of 5% CO₂ at 37°C for 3 days.

Three days later, 6 µg of pAAV-Venus, 6 µg of pRC2-mi342, and 12 µg of pHelper were mixed to prepare Liquid A. A solution including 72 µg of PEI (produced by Polysciences Inc.) was provided as Liquid B. Liquid A and Liquid B were mixed, and the resulting mixture was left to stand at room temperature for 15 minutes.

To the HEK293T cells on a fresh medium, the mixture of Liquid A and Liquid B was added. The resulting mixture was incubated in the atmosphere of 5% CO₂ at 37°C for 4 days.

Four days later, the fluid culture medium of the culture dish was collected. To the culture dish, 3 mL of a PBS solution was added, and then the PBS solution was collected. To the culture dish, 1 mL of an Accutase solution (produced by Innovative Cell Technologies, Inc.) was added to separate the cells, and the cell suspension liquid was collected. To the cell suspension liquid, 6 mL of a PBS solution was added, and then collected. The collected fluid was subjected to centrifugal separation to collect cells and a supernatant.

Measurement evaluation of the number of vector genomes of the obtained adeno-associated virus vector was carried out by real-time PCR in the same manner as in Example 2-8. The results are presented in Tables 1 and 2.

Regarding the results of Table 1, in Example 2-8, the adeno-associated virus vector of the Parvoviridae family was produced by transfecting the HEK293T cells with the three linear covalently closed DNAs obtained. In Example 2-9, moreover, the adeno-associated virus vector of the Parvoviridae family was produced by transfecting the HEK293T cells with the two linear covalently closed DNAs obtained.

With both the supernatant-derived samples and the cell-derived samples, the number of vector genomes of the target gene of the adeno-associated virus vector when the linear covalently closed DNAs were used (Examples 2-8 and 2-9) were equal to or higher than the number of vector genomes when the circular plasmid DNA was used (Comparative Example 1).

According to the results of Table 2, as the HEK293T cells were transfected with the circular plasmid DNA in Comparative Example 1, with the supernatant-derived samples, the number of vector genomes (2.02×10¹⁰ vg/dish) of the gene (ampR) encoding the antibiotic resistant protein (AmpR), which was the sequence (unnecessary sequence) other than the nucleic acid sequence encoding protein, was 14.4% relative to the number of vector genomes (1.40×10¹¹ vg/dish) of the nucleic acid sequence (target gene) encoding the target protein of Table 1. With the cell-derived samples, moreover, the number of the vector genomes (3.42×10⁹ vg/dish) of the sequence (unnecessary sequence) other than the nucleic acid sequence encoding a protein was 1.2% relative to the number of the vector genomes (2.75×10¹¹ vg/dish) of the nucleic acid sequence (target gene) encoding the protein of Table 1.

According to the results of Table 2, comparatively, the number of vector genomes of the sequences (unnecessary sequences) other than the nucleic acid sequences encoding proteins of the adeno-associated virus vectors (Examples 2-8 and 2-9) produced using the linear covalently closed DNAs were significantly reduced compared to the adeno-associated virus vector produced using the circular plasmid DNA (Comparative Example 1), and the vector genomes of the sequences (unnecessary sequences) other than the nucleic acid sequences encoding proteins were not detected at all (equal to or lower than the detection limit).

The results of Tables 1 and 2 indicated that, as the linear covalently closed DNAs were used as the vectors for transfecting the second host, the transfection efficiency to the second host increased due to the smaller size compared to the circular plasmid DNA, and the number of the vector genomes of the target genes of the adeno-associated virus vector of the Parvoviridae family increased. Moreover, the results indicated that, as the linear covalently closed DNAs did not include unnecessary base sequences, such as the nucleic acid sequence derived from the host, production of the high-quality adeno-associated virus vector of the Parvoviridae family was realized.

For example, embodiments of the present invention include the following.
<1> A vector including:
   a nucleic acid sequence encoding protelomerase;
   a pair of nucleic acid sequences recognized by the protelomerase; and
   a nucleic acid sequence located between the pair of nucleic acid sequences and encoding a protein.
<2> The vector according to <1>,
   wherein the vector is a vector for production of a linear covalently closed DNA.
<3> The vector according to <1> or <2>,
   wherein the vector is a double-stranded circular plasmid DNA vector.
<4> The vector according to any one of <1> to <3>, further including:
   a nucleic acid sequence regulating expression of the protelomerase.
<5> A linear covalently closed DNA production method, including:
   a gene introduction step of introducing a vector into a host, where the vector includes a nucleic acid sequence encoding protelomerase, a pair of nucleic acid sequences recognized by the protelomerase, and a nucleic acid sequence located between the pair of nucleic acid sequences and encoding a protein.
<6> The linear covalently closed DNA production method according to <5>,
   wherein the vector is a double-stranded circular plasmid DNA vector.
<7> The linear covalently closed DNA production method according to <5> or <6>,
   wherein the vector further includes a nucleic acid sequence regulating expression of the protelomerase.
<8> The linear covalently closed DNA production method according to <7>, further including,
   after the gene introduction step, an expression induction step of inducing expression of the protelomerase.
<9> A parvovirus vector production method, including:
   a gene introduction step of introducing, into a first host, a vector including a nucleic acid sequence encoding protelomerase, a pair of nucleic acid sequences recognized by the protelomerase, and a nucleic acid sequence located between the pair of nucleic acid sequences and encoding a protein; and
   a transfection step of transfecting a second host with a linear covalently closed DNA obtained in the gene introduction step.
<10> The parvovirus vector production method according to <9>, wherein the nucleic acid sequence encoding the protein is a nucleic acid sequence encoding a target protein to be introduced into the parvovirus vector, a nucleic acid sequence encoding a packaging protein, or a nucleic acid sequence encoding a helper protein.
<11> The parvovirus vector production method according to <9> or <10>,
   wherein the gene introduction step includes
      introducing, into the first host, a vector including the nucleic acid sequence encoding protelomerase, the pair of nucleic acid sequences recognized by the protelomerase, and a nucleic acid sequence located between the pair of nucleic acid sequences and encoding a target protein,
      introducing, into the first host, a vector including the nucleic acid sequence encoding protelomerase, the pair of nucleic acid sequences recognized by the protelomerase, and a nucleic acid sequence located between the pair of nucleic acid sequences and encoding a packaging protein, and
      introducing, into the first host, a vector including the nucleic acid sequence encoding protelomerase, the pair of nucleic acid sequences recognized by the protelomerase, and a nucleic acid sequence located between the pair of nucleic acid sequences and encoding a helper protein, and
   wherein the transfection step includes transfecting the second host with three linear covalently closed DNAs obtained in the gene introduction step, which are a linear covalently closed DNA including the nucleic acid sequence encoding the target protein, a linear covalently closed DNA including the nucleic acid sequence encoding the packaging protein, and a linear covalently closed DNA including the nucleic acid sequence encoding the helper protein.
<12> The parvovirus vector production method according to <9> or <10>,
   wherein the gene introduction step includes
      introducing, into the first host, a vector including the nucleic acid sequence encoding protelomerase, the pair of nucleic acid sequences recognized by the protelomerase, and a nucleic acid sequence located between the pair of nucleic acid sequence and encoding a target protein, and
      introducing, into the first host, a vector including the nucleic acid sequence encoding protelomerase, the pair of nucleic acid sequences recognized by the protelomerase, and a nucleic acid sequence located between the pair of nucleic acid sequences and encoding a packaging protein and a helper protein, and
   wherein the transfection step includes transfecting the second host with two linear covalently closed DNAs obtained in the gene introduction step, which are a linear covalently closed DNA including the nucleic acid sequence encoding the target protein, and a linear covalently closed DNA including the nucleic acid sequence encoding the packaging protein and the nucleic acid sequence encoding the helper protein.
<13> The parvovirus vector production method according to any one of <9> to <12>,
   wherein the vector is a double-stranded circular plasmid DNA vector.
<14> The parvovirus vector production method according to any one of <9> to <13>,
   wherein the vector further includes a nucleic acid sequence regulating expression of the protelomerase.
<15> The parvovirus vector production method according to <14>, further including:
   after the gene introduction step, an expression induction step of inducing expression of the protelomerase.
<16> The parvovirus vector production method according to any one of <10> to <15>,
   wherein the packaging protein includes Rep or Cap.
<17> The parvovirus vector production method according to any one of <10> to <15>,
   wherein the helper protein includes an adenovirus helper protein.
<18> The parvovirus vector production method according to any one of <9> to <17>,
   wherein in a culture supernatant of the second host transfected with the linear covalently closed DNA obtained in the gene introduction step, the number of vector genomes of sequences other than the nucleic acid sequence encoding the protein is 10% or less relative to the number of vector genomes of the nucleic acid sequence encoding the protein, as quantified by quantitative PCR, or
   in a cell solution of the second host transfected with the linear covalently closed DNA obtained in the gene introduction step, the number of vector genomes of sequences other than the nucleic acid sequence encoding the protein is 1% or less relative to the number of vector genomes of the nucleic acid sequence encoding the protein, as quantified by quantitative PCR.
<19> The parvovirus vector production method according to any one of <9> to <18>,
   wherein the parvovirus vector is an adeno-associated virus vector.
<20> A parvovirus-vector-producing cell,
   wherein the parvovirus vector is a parvovirus vector that is obtained using a linear covalently closed DNA and expresses a nucleic acid sequence encoding a protein, and
   wherein the number of vector genomes of sequences other than the nucleic acid sequence encoding the protein relative to the number of vector genomes of the nucleic acid sequence encoding the protein, as quantified by quantitative PCR, is 10% or less in a culture supernatant of the parvovirus-vector-producing cells, or 1% or less in a cell solution of the parvovirus-vector-producing cells.

## Claims

1. A vector comprising:
a nucleic acid sequence encoding protelomerase;
a pair of nucleic acid sequences recognized by the protelomerase; and
a nucleic acid sequence located between the pair of nucleic acid sequences and encoding a protein.

2. The vector according to claim 1,
wherein the vector is a vector for production of a linear covalently closed DNA.

3. The vector according to claim 1 or 2,
wherein the vector is a double-stranded circular plasmid DNA vector.

4. The vector according to any one of claims 1 to 3, further comprising:
a nucleic acid sequence regulating expression of the protelomerase.

5. A linear covalently closed DNA production method, comprising:
a gene introduction step of introducing a vector into a host, where the vector includes a nucleic acid sequence encoding protelomerase, a pair of nucleic acid sequences recognized by the protelomerase, and a nucleic acid sequence located between the pair of nucleic acid sequences and encoding a protein.

6. The linear covalently closed DNA production method according to claim 5,
wherein the vector is a double-stranded circular plasmid DNA vector.

7. The linear covalently closed DNA production method according to claim 5 or 6,
wherein the vector further includes a nucleic acid sequence regulating expression of the protelomerase.

8. The linear covalently closed DNA production method according to claim 7, further comprising,
after the gene introduction step, an expression induction step of inducing expression of the protelomerase.

9. A parvovirus vector production method, comprising:
a gene introduction step of introducing, into a first host, a vector including a nucleic acid sequence encoding protelomerase, a pair of nucleic acid sequences recognized by the protelomerase, and a nucleic acid sequence located between the pair of nucleic acid sequences and encoding a protein; and
a transfection step of transfecting a second host with a linear covalently closed DNA obtained in the gene introduction step.

10. The parvovirus vector production method according to claim 9,
wherein the nucleic acid sequence encoding the protein is a nucleic acid sequence encoding a target protein to be introduced into the parvovirus vector, a nucleic acid sequence encoding a packaging protein, or a nucleic acid sequence encoding a helper protein.

11. The parvovirus vector production method according to claim 9 or 10,
wherein the gene introduction step includes
introducing, into the first host, a vector including the nucleic acid sequence encoding protelomerase, the pair of nucleic acid sequences recognized by the protelomerase, and a nucleic acid sequence located between the pair of nucleic acid sequences and encoding a target protein,
introducing, into the first host, a vector including the nucleic acid sequence encoding protelomerase, the pair of nucleic acid sequences recognized by the protelomerase, and a nucleic acid sequence located between the pair of nucleic acid sequences and encoding a packaging protein, and
introducing, into the first host, a vector including the nucleic acid sequence encoding protelomerase, the pair of nucleic acid sequences recognized by the protelomerase, and a nucleic acid sequence located between the pair of nucleic acid sequences and encoding a helper protein, and
wherein the transfection step includes transfecting the second host with three linear covalently closed DNAs obtained in the gene introduction step, which are a linear covalently closed DNA including the nucleic acid sequence encoding the target protein, a linear covalently closed DNA including the nucleic acid sequence encoding the packaging protein, and a linear covalently closed DNA including the nucleic acid sequence encoding the helper protein.

12. The parvovirus vector production method according to claim 9 or 10,
wherein the gene introduction step includes
introducing, into the first host, a vector including the nucleic acid sequence encoding protelomerase, the pair of nucleic acid sequences recognized by the protelomerase, and a nucleic acid sequence located between the pair of nucleic acid sequence and encoding a target protein, and
introducing, into the first host, a vector including the nucleic acid sequence encoding protelomerase, the pair of nucleic acid sequences recognized by the protelomerase, and a nucleic acid sequence located between the pair of nucleic acid sequences and encoding a packaging protein and a helper protein, and
wherein the transfection step includes transfecting the second host with two linear covalently closed DNAs obtained in the gene introduction step, which are a linear covalently closed DNA including the nucleic acid sequence encoding the target protein, and a linear covalently closed DNA including the nucleic acid sequence encoding the packaging protein and the nucleic acid sequence encoding the helper protein.

13. The parvovirus vector production method according to any one of claims 9 to 12,
wherein the vector is a double-stranded circular plasmid DNA vector.

14. The parvovirus vector production method according to any one of claims 9 to 13,
wherein the vector further includes a nucleic acid sequence regulating expression of the protelomerase.

15. The parvovirus vector production method according to claim 14, further comprising:
after the gene introduction step, an expression induction step of inducing expression of the protelomerase.

16. The parvovirus vector production method according to any one of claims 10 to 15,
wherein the packaging protein includes Rep or Cap.

17. The parvovirus vector production method according to any one of claims 10 to 15,
wherein the helper protein includes an adenovirus helper protein.

18. The parvovirus vector production method according to any one of claims 9 to 17,
wherein in a culture supernatant of the second host transfected with the linear covalently closed DNA obtained in the gene introduction step, the number of vector genomes of sequences other than the nucleic acid sequence encoding the protein is 10% or less relative to the number of vector genomes of the nucleic acid sequence encoding the protein, as quantified by quantitative PCR, or
in a cell solution of the second host transfected with the linear covalently closed DNA obtained in the gene introduction step, the number of vector genomes of sequences other than the nucleic acid sequence encoding the protein is 1% or less relative to the number of vector genomes of the nucleic acid sequence encoding the protein, as quantified by quantitative PCR.

19. The parvovirus vector production method according to any one of claims 9 to 18,
wherein the parvovirus vector is an adeno-associated virus vector.

20. A parvovirus-vector-producing cell,
wherein the parvovirus vector is a parvovirus vector that is obtained using a linear covalently closed DNA and expresses a nucleic acid sequence encoding a protein, and
wherein the number of vector genomes of sequences other than the nucleic acid sequence encoding the protein relative to the number of vector genomes of the nucleic acid sequence encoding the protein, as quantified by quantitative PCR, is 10% or less in a culture supernatant of the parvovirus-vector-producing cells, or 1% or less in a cell solution of the parvovirus-vector-producing cells.
